# EUROPEAN PATENT APPLICATION

(11) **EP 4 667 050 A2**
(43) Date of publication of application: **24.12.2025**
(21) Application number: 25199386.1
(22) Date of filing: 31.05.2023
(51) Int. Cl.: A61P 3/00

(54) **MIGALASTAT FORMULATIONS HAVING IMPROVED PHARMACOKINETICS**

(30) Priority: 13.12.2022 US 202263432235 P; 11.05.2023 US 202318315928
(62) Divisional of application: 23176366.5
(71) Applicant: Amicus Therapeutics, Inc., Philadelphia, PA 19104 (US)
(72) Inventor: JOHNSON, Franklin, Philadelphia, PA, 19104 (US)
(74) Representative: Hoffmann Eitle

(57) **Abstract**

Provided are formulations comprising migalastat or a salt thereof having improved pharmacokineticsby limiting caffeine intake during migalastat therapy.

## Description

### TECHNICAL FIELD

Principles and embodiments of the present invention relate generally to migalastat formulations having improved pharmacokinetics.

### BACKGROUND

Many human diseases result from mutations that cause changes in the amino acid sequence of a protein which reduce its stability and may prevent it from folding properly. Proteins generally fold in a specific region of the cell known as the endoplasmic reticulum, or ER. The cell has quality control mechanisms that ensure that proteins are folded into their correct three-dimensional shape before they can move from the ER to the appropriate destination in the cell, a process generally referred to as protein trafficking. Misfolded proteins are often eliminated by the quality control mechanisms after initially being retained in the ER. In certain instances, misfolded proteins can accumulate in the ER before being eliminated. The retention of misfolded proteins in the ER interrupts their proper trafficking, and the resulting reduced biological activity can lead to impaired cellular function and ultimately to disease. In addition, the accumulation of misfolded proteins in the ER may lead to various types of stress on cells, which may also contribute to cellular dysfunction and disease.

Such mutations can lead to lysosomal storage disorders (LSDs), which are characterized by deficiencies of lysosomal enzymes due to mutations in the genes encoding the lysosomal enzymes. The resultant disease causes the pathologic accumulation of substrates of those enzymes, which include lipids, carbohydrates, and polysaccharides. Although there are many different mutant genotypes associated with each LSD, many of the mutations are missense mutations which can lead to the production of a less stable enzyme. These less stable enzymes are sometimes prematurely degraded by the ER-associated degradation pathway. This results in the enzyme deficiency in the lysosome, and the pathologic accumulation of substrate. Such mutant enzymes are sometimes referred to in the pertinent art as "folding mutants" or "conformational mutants."

Fabry disease, an LSD, is a progressive, X-linked inborn error of glycosphingolipid metabolism caused by a deficiency in the lysosomal enzyme α-galactosidase A (α-Gal A) as a result of mutations in the α-Gal A gene (GLA). Despite being an X-linked disorder, females can express varying degrees of clinical manifestations.

Fabry disease is classified by clinical manifestations into three groups: a classic form with generalized vasculopathy, an atypical variant form with clinical manifestations limited to cardiac tissue, and later-onset disease, which includes female carriers with mild to severe forms of the disease. The clinical manifestations include angiokeratoma (small, raised reddish-purple blemishes on the skin), acroparesthesias (burning in hands and feet), hypohidrosis (decreased ability to sweat), and characteristic corneal and lenticular opacities (The Metabolic and Molecular Bases of Inherited Disease, 8th Edition 2001, Scriver et al., ed., pp. 3733-3774, McGraw-Hill, New York).

Fabry is a rare disease with incidence estimated between 1 in 40,000 males to 1 in 117,000 in the general population. Moreover, there are variants of later onset phenotype of Fabry disease that can be under-diagnosed, as they do not present with classical signs and symptoms. This, and newborn screening for Fabry disease, suggests that the actual incidence of Fabry disease can be higher than currently estimated.

Untreated, life expectancy in Fabry patients is reduced and death usually occurs in the fourth or fifth decade because of vascular disease affecting the kidneys, heart and/or central nervous system. The enzyme deficiency leads to intracellular accumulation of the substrate, globotriaosylceramide (GL-3) in the vascular endothelium and visceral tissues throughout the body. The heart may also become enlarged and the kidneys may become progressively involved. Gradual deterioration of renal function and the development of azotemia, due to glycosphingolipid deposition, usually occur in the third to fifth decades of life, but can occur as early as in the second decade. Renal lesions are found in both hemizygous (male) and heterozygous (female) patients. The affected male's life expectancy is reduced, and death usually occurs in the fourth or fifth decade as a result of vascular disease of the heart, brain, and/or kidneys. Other symptoms include fever and gastrointestinal difficulties, particularly after eating.

Cardiac disease as a result of Fabry disease occurs in most males and many females. Early cardiac findings include left ventricular enlargement, valvular involvement and conduction abnormalities. Mitral insufficiency is the most frequent valvular lesion typically present in childhood or adolescence. Cerebrovascular manifestations result primarily from multifocal small-vessel involvement and can include thromboses, transient ischemic attacks, basilar artery ischemia and aneurysm, seizures, hemiplegia, hemianesthesia, aphasia, labyrinthine disorders, or cerebral hemorrhages. Average age of onset of cerebrovascular manifestations is 33.8 years. Personality change and psychotic behavior can manifest with increasing age.

Individuals with later-onset Fabry disease can be male or female. Late-onset Fabry disease presents as the atypical variant form, and growing evidence indicates there may be a significant number of "atypical variants" which are unaccounted for in the world. Females, who inherit an X chromosome containing an a-GAL mutation, may exhibit symptoms later in life, significantly increasing the prevalence of this disease. These patients typically first experience disease symptoms in adulthood, and often have disease symptoms focused on a single organ. For example, many males and females with later-onset Fabry disease have enlargement of the left ventricle of the heart. Later-onset Fabry disease may also present in the form of strokes of unknown cause. As the patients advance in age, the cardiac complications of the disease progress, and can lead to death.

Patients with the milder "cardiac variant" of Fabry disease normally have 5-15% of normal a-GAL activity, and present with left ventricular hypertrophy or a cardiomyopathy. These cardiac variant patients remain essentially asymptomatic when their classically affected counterparts are severely compromised. Cardiac variants were found in 1 1% of adult male patients with unexplained left ventricular hypertrophic cardiomyopathy, suggesting that Fabry disease may be more frequent than previously estimated (Nakao et al., N. Engl. J. Med. 1995; 333: 288-293).

There have been several approaches to treatment of Fabry disease. One approved therapy for treating Fabry disease is enzyme replacement therapy (ERT), which typically involves intravenous, infusion of a purified form of the corresponding wild-type protein (Fabrazyme^{®}, Genzyme Corp.). ERT has several drawbacks, however. One of the main complications with enzyme replacement therapy is rapid degradation of the infused protein, which leads to the need for numerous, costly high dose infusions. ERT has several additional caveats, such as difficulties with large-scale generation, purification, and storage of properly folded protein; obtaining glycosylated native protein; generation of an anti-protein immune response; and inability of protein to cross the blood-brain barrier to mitigate central nervous system pathologies (i.e., low bioavailability). In addition, replacement enzyme cannot penetrate the heart or kidney in sufficient amounts to reduce substrate accumulation in the renal podocytes or cardiac myocytes, which figure prominently in Fabry pathology.

Additionally, ERT typically involves intravenous, infusion of a purified form of the corresponding wild-type protein. Two α-Gal A products are currently available for the treatment of Fabry disease: agalsidase alfa (Replagal^{®}, Shire Human Genetic Therapies) and agalsidase beta (Fabrazyme^{®}; Sanofi Genzyme Corporation). While ERT is effective in many settings, the treatment also has limitations. ERT has not been demonstrated to decrease the risk of stroke, cardiac muscle responds slowly, and GL-3 elimination from some of the cell types of the kidneys is limited. Some patients also develop immune reactions to ERT.

Another approach to treating some enzyme deficiencies involves the use of small molecule inhibitors to reduce production of the natural substrate of deficient enzyme proteins, thereby ameliorating the pathology. This "substrate reduction" approach has been specifically described for a class of about 40 related enzyme disorders called lysosomal storage disorders that include glycosphingolipid storage disorders. The small molecule inhibitors proposed for use as therapy are specific for inhibiting the enzymes involved in synthesis of glycolipids, reducing the amount of cellular glycolipid that needs to be broken down by the deficient enzyme.

A third approach to treating Fabry disease has been treatment with what are called pharmacological chaperones (PCs). Such PCs include small molecule inhibitors of α-Gal A, which can bind to the α-Gal A to increase the stability of both mutant enzyme and the corresponding wild type. One such PC for α-Gal A is migalastat.

Accordingly, there remains a need for therapies for the treatment of Fabry disease.

### SUMMARY

Various aspects of the present invention relate to migalastat formulations having improved pharmacokinetics.

One aspect of the present invention pertains to a method of administering migalastat to a patient, the method comprising orally administering to the patient a formulation comprising a therapeutically effective dose of migalastat or a salt thereof, wherein the patient does not consume caffeine within a certain time interval of administering the formulation comprising migalastat or a salt thereof. In various embodiments, this time interval includes abstaining from caffeine for at least 30 minutes, at least 60 minutes (1 hour), at least 90 minutes (1.5 hours), at least 2 hours, at least 2.5 hours, at least 3 hours or at least 4 hours prior to administering the migalastat or salt thereof and at least 30 minutes, at least 60 minutes (1 hour), at least 90 minutes (1.5 hours), at least 2 hours, at least 2.5 hours, at least 3 hours or at least 4 hours after administering the migalastat or salt thereof.

In some embodiments, the patient does not consume caffeine within a time interval from at least 1 hour prior to and at least 1 hour after administering the migalastat or salt thereof, i.e. the patient does not consume caffeine within about 1 hour of administering the formulation comprising migalastat or a salt thereof.

In some embodiments, the patient does not consume caffeine within a time interval from at least 2 hours prior to and at least 1 hour after administering the migalastat or salt thereof.

In some embodiments, the patient does not consume caffeine within a time interval from at least 2 hours prior to and at least 2 hours after administering the migalastat or salt thereof, i.e. the patient does not consume caffeine within about 2 hours of administering the formulation comprising migalastat or a salt thereof.

In some embodiments, the patient does not consume caffeine within a time interval from at least 3 hours prior to and at least 2 hours after administering the migalastat or salt thereof.

In some embodiments, the patient does not consume caffeine within a time interval from at least 3 hours prior to and at least 3 hours after administering the migalastat or salt thereof, i.e. the patient does not consume caffeine within about 3 hours of administering the formulation comprising migalastat or a salt thereof.

In some embodiments, the patient consumes caffeine outside of the time interval for abstaining from caffeine. For example, if the time interval for abstaining from caffeine is at least 2 hours prior to and at least 2 hours after administering the migalastat or salt thereof, then in some embodiments the patient consumes caffeine at least 2 hours prior to and/or at least 2 hours after administering the migalastat or salt thereof. In various embodiments, the patient consumes caffeine at least 30 minutes, at least 60 minutes (1 hour), at least 90 minutes (1.5 hours), at least 2 hours, at least 2.5 hours, at least 3 hours or at least 4 hours prior to administering the migalastat or salt thereof. In various embodiments, the patient consumes caffeine at least 30 minutes, at least 60 minutes (1 hour), at least 90 minutes (1.5 hours), at least 2 hours, at least 2.5 hours, at least 3 hours or at least 4 hours after administering the migalastat or salt thereof.

In some embodiments, not consuming caffeine within a certain time interval of administering the formulation comprising migalastat or a salt thereof provides improvements in the pharmacokinetics of migalastat, such as avoiding a decrease in migalastat area under the curve (AUC) and/or maximum plasma concentration (Cₘₐₓ). In some embodiments, the patient does not consume caffeine within 2 hours of administering the formulation comprising migalastat or a salt thereof to avoid a decrease in AUC and Cₘₐₓ for migalastat of about 57% and about 60%, respectively.

In some embodiments, the patient fasts during the time interval for abstaining from caffeine. In some embodiments, the patient does not consume food for at least 2 hours before and at least 2 hours after administering the migalastat or salt thereof and the patient does not consume caffeine for at least 2 hours before and at least 2 hours after administering the migalastat or salt thereof.

In some embodiments, the patient fasts for a different time interval than the time interval for abstaining from caffeine.

In some embodiments, the therapeutically effective dose of migalastat or a salt thereof is in a range of from about 100 mg to about 150 mg every other day.

In some embodiments, the therapeutically effective dose of migalastat or a salt thereof is about 123 mg free base equivalent (FBE) every other day.

In some embodiments, the therapeutically effective dose of migalastat or a salt thereof is about 150 mg of migalastat hydrochloride every other day.

In one or more embodiments, the formulation comprises an oral dosage form. In some embodiments, the oral dosage form comprises a tablet, a capsule or a solution.

Another aspect of the present invention pertains to a method of treatment of Fabry disease in a human patient in need thereof, the method comprising orally administering to the patient a formulation comprising a therapeutically effective dose of migalastat or a salt thereof, wherein the patient does not consume caffeine within a certain time interval of administering the formulation comprising migalastat or a salt thereof. This method of treatment can have any of the features described herein related to the methods of administering migalastat.

In one or more embodiments, the patient has a HEK assay amenable mutation in α-galactosidase A. In one or more embodiments, the mutation is disclosed in a pharmacological reference table. In one or more embodiments, the pharmacological reference table is provided in a product label for a migalastat product approved for the treatment of Fabry disease. In one or more embodiments, the pharmacological reference table is provided in a product label for GALAFOLD^{®}. In one or more embodiments, the pharmacological reference table is provided at a website. In one or more embodiments, the website is one or more of www.galafoldamenabilitytable.com or www.fabrygenevariantsearch.com.

### BRIEF DESCRIPTION OF THE DRAWINGS

Further features of the present invention will become apparent from the following written description and the accompanying figures, in which:
FIGS. 1A-E show the full DNA sequence of the human wild-type GLA gene (SEQ ID NO: 1);
FIG. 2 shows the wild-type α-Gal A protein (SEQ ID NO: 2);
FIG. 3 shows the nucleic acid sequence encoding the wild-type α-Gal A protein (SEQ ID NO: 3);
FIG. 4 shows the study schematic for a study investigating the effect of caffeine and sweeteners on migalastat pharmacokinetics; and
FIG. 5 shows the migalastat concentration-time profiles when administered with caffeine and various sweeteners.

### DETAILED DESCRIPTION

Before describing several exemplary embodiments of the invention, it is to be understood that the invention is not limited to the details of construction or process steps set forth in the following description. The invention is capable of other embodiments and of being practiced or being carried out in various ways.

Various aspects of the present invention pertain to the administration of migalastat such as for the treatment of Fabry disease. It has surprisingly been discovered that the co-administration of caffeine with migalastat has a negative effect on the pharmacokinetics of migalastat, independent of the food effect on migalastat pharmacokinetics. Accordingly, various embodiments of the present invention relate to the administration of migalastat or salt thereof without the concurrent administration of caffeine, i.e. the patient does not consume caffeine within a certain time interval of administering the migalastat or salt thereof.

### Definitions

The terms used in this specification generally have their ordinary meanings in the art, within the context of this invention and in the specific context where each term is used. Certain terms are discussed below, or elsewhere in the specification, to provide additional guidance to the practitioner in describing the compositions and methods of the invention and how to make and use them.

As used herein, the phrase "the patient does not consume caffeine" and similar language refers to the patient not consuming (e.g. eating or drinking) food, beverages or other products that contain caffeine. In some embodiments, a food, beverage or product is considered to be caffeine-containing if it includes a certain amount of caffeine, such as more than 1 mg, 2 mg, 5 mg or 10 mg of caffeine. In some embodiments, examples of caffeine-containing beverages include coffee, espresso, tea, caffeinated energy drinks and caffeinated sodas.

The term "Fabry disease" refers to an X-linked inborn error of glycosphingolipid catabolism due to deficient lysosomal α-Gal A activity. This defect causes accumulation of the substrate globotriaosylceramide ("GL-3", also known as Gb3 or ceramide trihexoside) and related glycosphingolipids in vascular endothelial lysosomes of the heart, kidneys, skin, and other tissues. Another substrate of the enzyme is plasma globotriaosylsphingosine ("plasma lyso-Gb₃").

The term "atypical Fabry disease" refers to patients with primarily cardiac manifestations of the α-Gal A deficiency, namely progressive GL-3 accumulation in myocardial cells that leads to significant enlargement of the heart, particularly the left ventricle.

A "carrier" is a female who has one X chromosome with a defective α-Gal A gene and one X chromosome with the normal gene and in whom X chromosome inactivation of the normal allele is present in one or more cell types. A carrier is often diagnosed with Fabry disease.

A "patient" refers to a subject who has been diagnosed with or is suspected of having a particular disease. The patient may be human or animal.

A "Fabry patient" refers to an individual who has been diagnosed with or suspected of having Fabry disease and has a mutated α-Gal A as defined further below. Characteristic markers of Fabry disease can occur in male hemizygotes and female carriers with the same prevalence, although females typically are less severely affected.

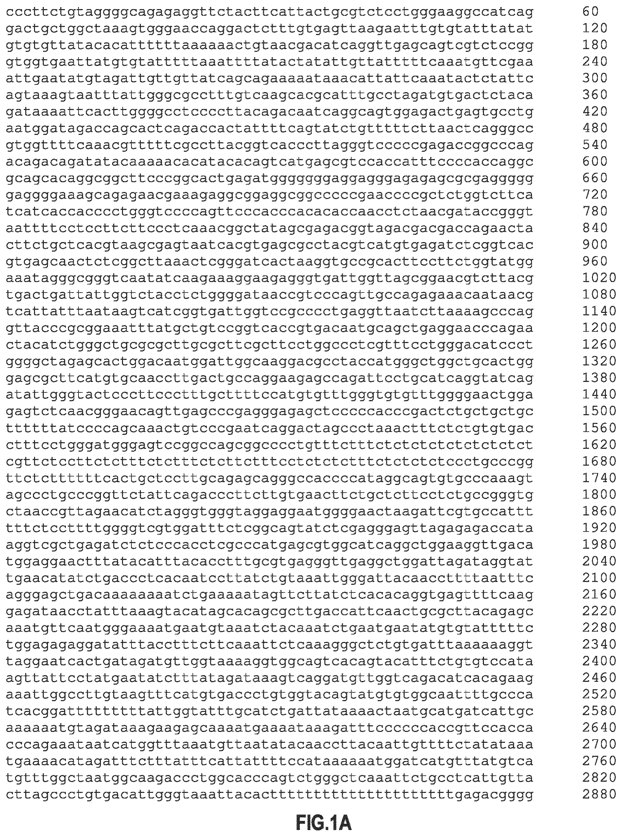

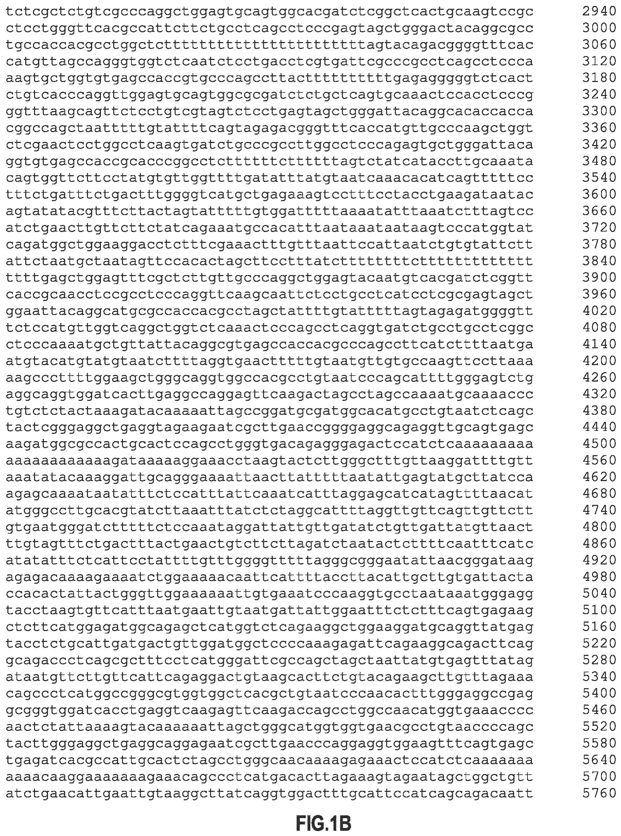

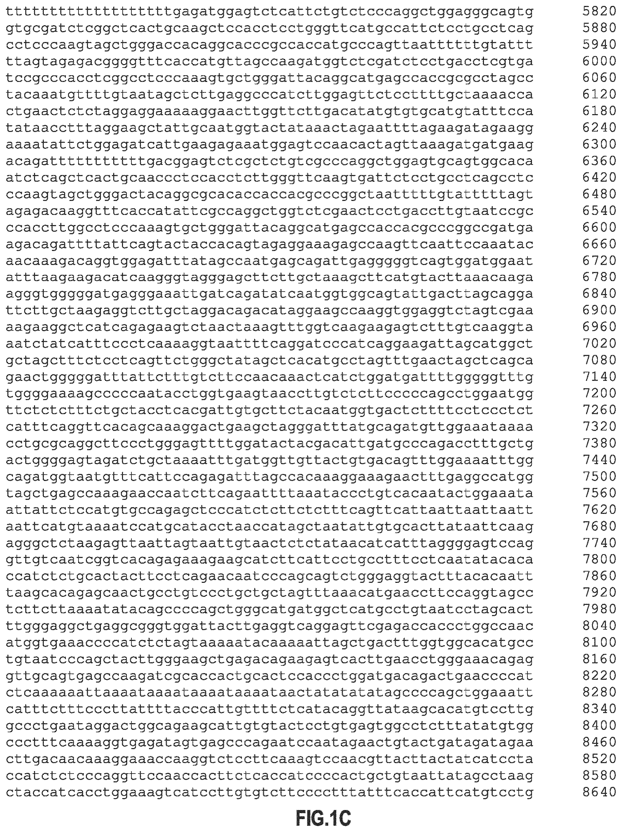

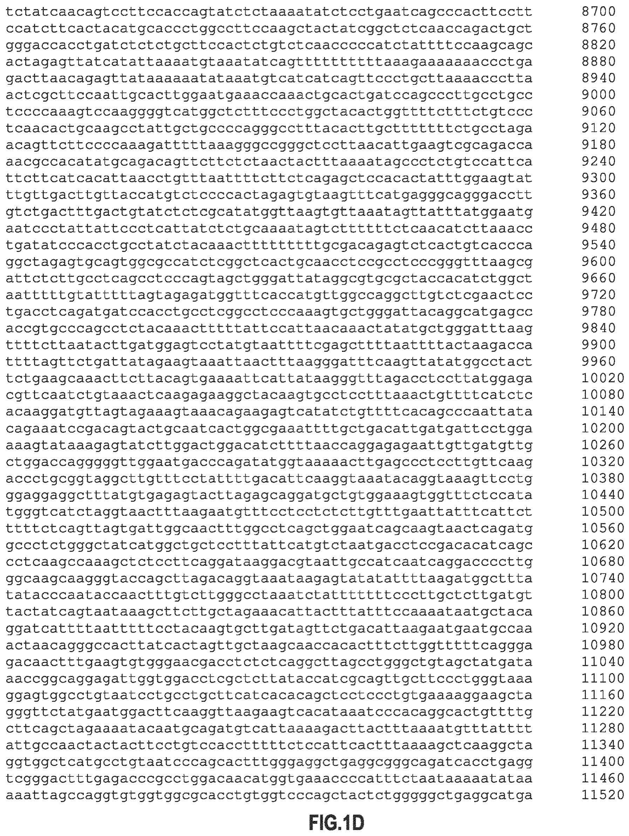

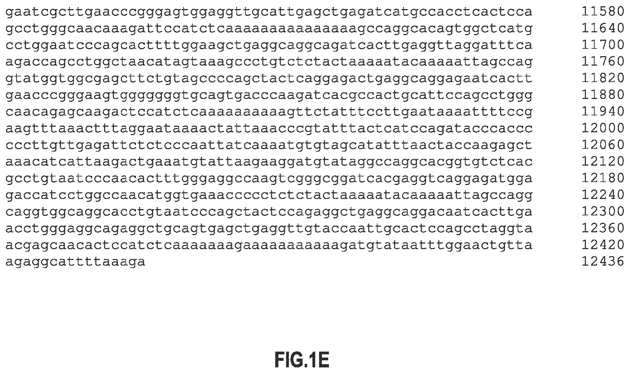

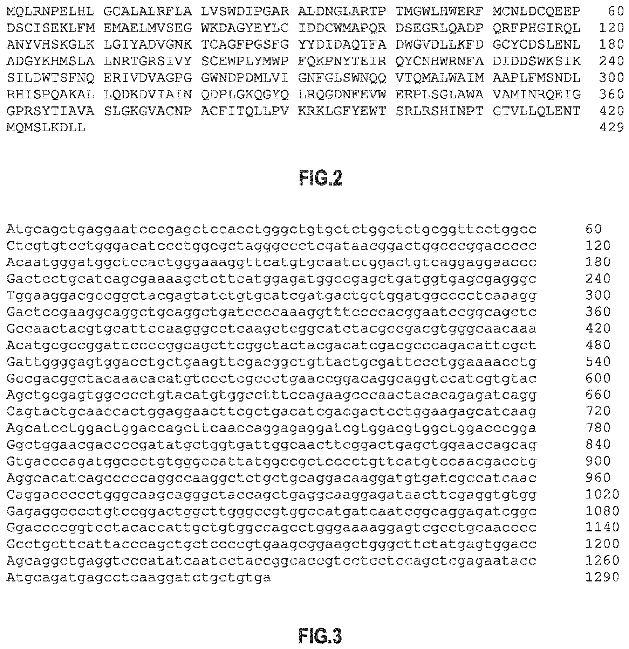

Human α-galactosidase A (α-Gal A) refers to an enzyme encoded by the human GLA gene. The full DNA sequence of α-Gal A, including introns and exons, is available in GenBank Accession No. X14448.1 and shown in FIG. 1A-E (SEQ ID NO: 1). The human α-Gal A enzyme consists of 429 amino acids and is available in GenBank Accession Nos. X14448.1 and U78027.1 and shown in FIG. 2 (SEQ ID NO: 2). The nucleic acid sequence that only includes the coding regions (i.e. exons) of SEQ ID NO: 1 is shown in FIG. 3 (SEQ ID NO: 3).

The term "mutant protein" includes a protein which has a mutation in the gene encoding the protein which results in the inability of the protein to achieve a stable conformation under the conditions normally present in the endoplasmic reticulum (ER). The failure to achieve a stable conformation results in a substantial amount of the enzyme being degraded, rather than being transported to the lysosome. Such a mutation is sometimes called a "conformational mutant." Such mutations include, but are not limited to, missense mutations, and in-frame small deletions and insertions.

As used herein in one embodiment, the term "mutant α-Gal A" includes an α-Gal A which has a mutation in the gene encoding α-Gal A which results in the inability of the enzyme to achieve a stable conformation under the conditions normally present in the ER. The failure to achieve a stable conformation results in a substantial amount of the enzyme being degraded, rather than being transported to the lysosome.

As used herein, the term "pharmacological chaperone" ("PC") or "specific pharmacological chaperone" ("SPC") refers to any molecule including a small molecule, protein, peptide, nucleic acid, carbohydrate, etc. that specifically binds to a protein and has one or more of the following effects: (i) enhances the formation of a stable molecular conformation of the protein; (ii) induces trafficking of the protein from the ER to another cellular location, preferably a native cellular location, *i.e.*, prevents ER-associated degradation of the protein; (iii) prevents aggregation of misfolded proteins; and/or (iv) restores or enhances at least partial wild-type function and/or activity to the protein. A compound that specifically binds to *e.g.,* α-Gal A, means that it binds to and exerts a chaperone effect on the enzyme and not a generic group of related or unrelated enzymes. More specifically, this term does not refer to endogenous chaperones, such as BiP, or to non-specific agents which have demonstrated non-specific chaperone activity against various proteins, such as glycerol, DMSO or deuterated water, *i.e.,* chemical chaperones. In one or more embodiments of the present invention, the PC may be a reversible competitive inhibitor. In one embodiment, the PC is migalastat or a salt thereof. In another embodiment, the PC is migalastat free base (*e.g.*, 123 mg of migalastat free base). In yet another embodiment, the PC is a salt of migalastat (*e.g.*, 150 mg of migalastat HCl).

A "competitive inhibitor" of an enzyme can refer to a compound which structurally resembles the chemical structure and molecular geometry of the enzyme substrate to bind the enzyme in approximately the same location as the substrate. Thus, the inhibitor competes for the same active site as the substrate molecule, thus increasing the Km. Competitive inhibition is usually reversible if sufficient substrate molecules are available to displace the inhibitor, *i.e.*, competitive inhibitors can bind reversibly. Therefore, the amount of enzyme inhibition depends upon the inhibitor concentration, substrate concentration, and the relative affinities of the inhibitor and substrate for the active site.

As used herein, the term "specifically binds" refers to the interaction of a pharmacological chaperone with a protein such as α-Gal A, specifically, an interaction with amino acid residues of the protein that directly participate in contacting the pharmacological chaperone. A pharmacological chaperone specifically binds a target protein, *e.g.*, α-Gal A, to exert a chaperone effect on the protein and not a generic group of related or unrelated proteins. The amino acid residues of a protein that interact with any given pharmacological chaperone may or may not be within the protein's "active site." Specific binding can be evaluated through routine binding assays or through structural studies, *e.g.*, co-crystallization, NMR, and the like. The active site for α-Gal A is the substrate binding site.

"Deficient α-Gal A activity" refers to α-Gal A activity in cells from a patient which is below the normal range as compared (using the same methods) to the activity in normal individuals not having or suspected of having Fabry or any other disease (especially a blood disease).

As used herein, the terms "enhance α-Gal A activity" or "increase α-Gal A activity" refer to increasing the amount of α-Gal A that adopts a stable conformation in a cell contacted with a pharmacological chaperone specific for the α-Gal A, relative to the amount in a cell (preferably of the same cell-type or the same cell, *e.g.*, at an earlier time) not contacted with the pharmacological chaperone specific for the α-Gal A. This term also refers to increasing the trafficking of α-Gal A to the lysosome in a cell contacted with a pharmacological chaperone specific for the α-Gal A, relative to the trafficking of α-Gal A not contacted with the pharmacological chaperone specific for the protein. These terms refer to both wild-type and mutant α-Gal A. In one embodiment, the increase in the amount of α-Gal A in the cell is measured by measuring the hydrolysis of an artificial substrate in lysates from cells that have been treated with the PC. An increase in hydrolysis is indicative of increased α-Gal A activity.

The term "α-Gal A activity" refers to the normal physiological function of a wild-type α-Gal A in a cell. For example, α-Gal A activity includes hydrolysis of GL-3.

A "responder" is an individual diagnosed with or suspected of having a lysosomal storage disorder (LSD), such, for example Fabry disease, whose cells exhibit sufficiently increased α-Gal A activity, respectively, and/or amelioration of symptoms or enhancement in surrogate markers, in response to contact with a PC. Non-limiting examples of enhancements in surrogate markers for Fabry are lyso-GB3 and those disclosed in US Patent Application Publication No. U.S. 2010/0113517, which is hereby incorporated by reference in its entirety.

Non-limiting examples of improvements in surrogate markers for Fabry disease disclosed in U.S. 2010/0113517 include increases in α-Gal A levels or activity in cells (*e.g.*, fibroblasts) and tissue; reductions in of GL-3 accumulation; decreased plasma concentrations of homocysteine and vascular cell adhesion molecule-1 (VCAM-1); decreased GL-3 accumulation within myocardial cells and valvular fibrocytes; reduction in plasma lyso-Gb₃; reduction in cardiac hypertrophy (especially of the left ventricle), amelioration of valvular insufficiency, and arrhythmias; amelioration of proteinuria; decreased urinary concentrations of lipids such as CTH, lactosylceramide, ceramide, and increased urinary concentrations of glucosylceramide and sphingomyelin; the absence of laminated inclusion bodies (Zebra bodies) in glomerular epithelial cells; improvements in renal function; mitigation of hypohidrosis; the absence of angiokeratomas; and improvements in hearing abnormalities such as high frequency sensorineural hearing loss progressive hearing loss, sudden deafness, or tinnitus. Improvements in neurological symptoms include prevention of transient ischemic attack (TIA) or stroke; and amelioration of neuropathic pain manifesting itself as acroparaesthesia (burning or tingling in extremities). Another type of clinical marker that can be assessed for Fabry disease is the prevalence of deleterious cardiovascular manifestations. Common cardiac-related signs and symptoms of Fabry disease include left ventricular hypertrophy, valvular disease (especially mitral valve prolapse and/or regurgitation), premature coronary artery disease, angina, myocardial infarction, conduction abnormalities, arrhythmias, congestive heart failure.

The dose that achieves one or more of the aforementioned responses is a "therapeutically effective dose."

The phrase "pharmaceutically acceptable" refers to molecular entities and compositions that are physiologically tolerable and do not typically produce untoward reactions when administered to a human. In some embodiments, as used herein, the term "pharmaceutically acceptable" means approved by a regulatory agency of the Federal or a state government or listed in the U.S. Pharmacopoeia or other generally recognized pharmacopoeia for use in animals, and more particularly in humans. The term "carrier" in reference to a pharmaceutical carrier refers to a diluent, adjuvant, excipient, or vehicle with which the compound is administered. Such pharmaceutical carriers can be sterile liquids, such as water and oils. Water or aqueous solution saline solutions and aqueous dextrose and glycerol solutions are preferably employed as carriers, particularly for injectable solutions. Suitable pharmaceutical carriers are described in "Remington's Pharmaceutical Sciences" by E. W. Martin, 18th Edition, or other editions.

As used herein, the term "isolated" means that the referenced material is removed from the environment in which it is normally found. Thus, an isolated biological material can be free of cellular components, *i.e.*, components of the cells in which the material is found or produced. In the case of nucleic acid molecules, an isolated nucleic acid includes a PCR product, an mRNA band on a gel, a cDNA, or a restriction fragment. In another embodiment, an isolated nucleic acid is preferably excised from the chromosome in which it may be found, and more preferably is no longer joined to non-regulatory, non-coding regions, or to other genes, located upstream or downstream of the gene contained by the isolated nucleic acid molecule when found in the chromosome. In yet another embodiment, the isolated nucleic acid lacks one or more introns. Isolated nucleic acids include sequences inserted into plasmids, cosmids, artificial chromosomes, and the like. Thus, in a specific embodiment, a recombinant nucleic acid is an isolated nucleic acid. An isolated protein may be associated with other proteins or nucleic acids, or both, with which it associates in the cell, or with cellular membranes if it is a membrane-associated protein. An isolated organelle, cell, or tissue is removed from the anatomical site in which it is found in an organism. An isolated material may be, but need not be, purified.

The term "enzyme replacement therapy" or "ERT" refers to the introduction of a non-native, purified enzyme into an individual having a deficiency in such enzyme. The administered protein can be obtained from natural sources or by recombinant expression (as described in greater detail below). The term also refers to the introduction of a purified enzyme in an individual otherwise requiring or benefiting from administration of a purified enzyme, e.g., suffering from enzyme insufficiency. The introduced enzyme may be a purified, recombinant enzyme produced *in vitro,* or protein purified from isolated tissue or fluid, such as, *e.g.*, placenta or animal milk, or from plants.

The term "ERT-naïve patient" refers to a Fabry patient that has never received ERT or has not received ERT for at least 6 months prior to initiating migalastat therapy.

The term "ERT-experienced patient" refers to a Fabry patient that was receiving ERT immediately prior to initiating migalastat therapy. In some embodiments, the ERT-experienced patient has received at least 12 months of ERT immediately prior to initiating migalastat therapy.

As used herein, the term "free base equivalent" or "FBE" refers to the amount of migalastat present in the migalastat or salt thereof. In other words, the term "FBE" means either an amount of migalastat free base, or the equivalent amount of migalastat free base that is provided by a salt of migalastat. For example, due to the weight of the hydrochloride salt, 150 mg of migalastat hydrochloride only provides as much migalastat as 123 mg of the free base form of migalastat. Other salts are expected to have different conversion factors, depending on the molecular weight of the salt.

The term "migalastat" encompasses migalastat free base or a pharmaceutically acceptable salt thereof (*e.g.*, migalastat HCl), unless specifically indicated to the contrary.

The terms "mutation" and "variant" (*e.g*., as in "amenable mutation or variant") refer to a change in the nucleotide sequence of a gene or a chromosome. The two terms referred herein are typically used together - *e.g.*, as in "mutation or variant"- referring to the change in nucleotide sequence stated in the previous sentence. If only one of the two terms is recited for some reason, the missing term was intended to be included and one should understand as such. Furthermore, the terms "amenable mutation" and "amenable variant" refer to a mutation or variant that is amenable to PC therapy, e.g., a mutation that is amenable to migalastat therapy. A particular type of amenable mutation or variant is a "HEK assay amenable mutation or variant", which is a mutation or variant that is determined to be amenable to migalastat therapy according to the criteria in the *in vitro* HEK assay described herein and in U.S. Patent No. 8,592,362, which is hereby incorporated by reference in its entirety.

The terms "about" and "approximately" shall generally mean an acceptable degree of error for the quantity measured given the nature or precision of the measurements. Typical, exemplary degrees of error are within 20 percent (%), preferably within 10%, and more preferably within 5% of a given value or range of values. Alternatively, and particularly in biological systems, the terms "about" and "approximately" may mean values that are within an order of magnitude, preferably within 10- or 5-fold, and more preferably within 2-fold of a given value. Numerical quantities given herein are approximate unless stated otherwise, meaning that the term "about" or "approximately" can be inferred when not expressly stated.

### Fabry Disease

Fabry disease is a rare, progressive and devastating X-linked lysosomal storage disorder (LSD). Mutations in the GLA gene result in a deficiency of the lysosomal enzyme, α-Gal A, which is required for glycosphingolipid metabolism. Beginning early in life, the reduction in α-Gal A activity results in an accumulation of glycosphingolipids, including GL-3 and plasma lyso-Gb3, and leads to the symptoms and life-limiting sequelae of Fabry disease, including pain, gastrointestinal symptoms, renal failure, cardiomyopathy, cerebrovascular events, and early mortality. Early initiation of therapy and lifelong treatment provide an opportunity to slow disease progression and prolong life expectancy.

Fabry disease encompasses a spectrum of disease severity and age of onset, although it has traditionally been divided into 2 main phenotypes, "classic" and "late-onset". The classic phenotype has been ascribed primarily to males with undetectable to low α-Gal A activity and earlier onset of renal, cardiac and/or cerebrovascular manifestations. The late-onset phenotype has been ascribed primarily to males with higher residual α-Gal A activity and later onset of these disease manifestations. Heterozygous female carriers typically express the late-onset phenotype but depending on the pattern of X-chromosome inactivation may also display the classic phenotype.

More than 1,000 Fabry disease-causing GLA mutations have been identified. The GLA mutation includes but not limited to missense, nonsense, and splicing mutations, in addition to small deletions and insertions, and larger gene rearrangements. Approximately 60% are missense mutations, resulting in single amino acid substitutions in the α-Gal A enzyme. Missense GLA mutations often result in the production of abnormally folded and unstable forms of α-Gal A and the majority are associated with the classic phenotype. Normal cellular quality control mechanisms in the ER block the transit of these abnormal proteins to lysosomes and target them for premature degradation and elimination. Many missense mutant forms are targets for migalastat, an α-Gal A-specific pharmacological chaperone.

The clinical manifestations of Fabry disease span a broad spectrum of severity and roughly correlate with a patient's residual α-Gal A levels. The majority of currently treated patients are referred to as classic Fabry patients, most of whom are males. These patients experience disease of various organs, including the kidneys, heart and brain, with disease symptoms first appearing in adolescence and typically progressing in severity until death in the fourth or fifth decade of life. A number of recent studies suggest that there are a large number of undiagnosed males and females that have a range of Fabry disease symptoms, such as impaired cardiac or renal function and strokes, that usually first appear in adulthood. Individuals with this type of Fabry disease, referred to as later-onset Fabry disease, tend to have higher residual α-Gal A levels than classic Fabry patients. Individuals with later-onset Fabry disease typically first experience disease symptoms in adulthood, and often have disease symptoms focused on a single organ, such as enlargement of the left ventricle or progressive kidney failure. In addition, later-onset Fabry disease may also present in the form of strokes of unknown cause.

Because Fabry disease is rare, involves multiple organs, has a wide age range of onset, and is heterogeneous, proper diagnosis is a challenge. For example, Fabry patients have progressive kidney impairment, and untreated patients exhibit end-stage renal impairment by the fifth decade of life. Deficiency in α-Gal A activity leads to accumulation of globotriaosylceramide (Gb3) and related glycosphingolipids in many cell types including cells in the kidney. Gb3 accumulates in podocytes, epithelial cells and the tubular cells of the distal tubule and loop of Henle. Impairment in kidney function can manifest as proteinuria and reduced glomerular filtration rate.

Furthermore, awareness is low among health care professionals and misdiagnoses are frequent. Diagnosis of Fabry disease is most often confirmed on the basis of decreased α-Gal A activity in plasma or peripheral leukocytes (WBCs) once a patient is symptomatic, coupled with mutational analysis. In females, diagnosis is even more challenging since the enzymatic identification of carrier females is less reliable due to random X-chromosomal inactivation in some cells of carriers. For example, some obligate carriers (daughters of classically affected males) have α-Gal A enzyme activities ranging from normal to very low activities. Since carriers can have normal α-Gal A enzyme activity in leukocytes, only the identification of an α-Gal A mutation by genetic testing provides precise carrier identification and/or diagnosis.

In one or more embodiments, mutant forms of α-Gal A are considered to be amenable to migalastat are defined as showing a relative increase (+10 µM migalastat) of ≥1.20-fold and an absolute increase (+ 10 µM migalastat) of ≥ 3.0% wild-type (WT) when the mutant form of α-Gal A is expressed in HEK-293 cells (referred to as the "HEK assay") according to Good Laboratory Practice (GLP)-validated *in vitro* assay (GLP HEK or Migalastat Amenability Assay). Such mutations are also referred to herein as "HEK assay amenable" mutations.

Previous screening methods have been provided that assess enzyme enhancement prior to the initiation of treatment. For example, an assay using HEK-293 cells has been utilized in clinical trials to predict whether a given mutation will be responsive to pharmacological chaperone (*e.g.*, migalastat) treatment. In this assay, cDNA constructs are created. The corresponding α-Gal A mutant forms are transiently expressed in HEK-293 cells. Cells are then incubated ± migalastat (17 nM to 1 mM) for 4 to 5 days. After, α-Gal A levels are measured in cell lysates using a synthetic fluorogenic substrate (4-MU-α-Gal) or by western blot. This has been done for known disease-causing missense or small in-frame insertion/deletion mutations. Mutations that have previously been identified as responsive to a PC (*e.g.*, migalastat) using these methods are listed in U.S. Patent No. 8,592,362, which is hereby incorporated by reference in its entirety.

### Pharmacological Chaperones

The binding of small molecule inhibitors of enzymes associated with LSDs can increase the stability of both mutant enzyme and the corresponding wild-type enzyme (see U.S. Pat. Nos. 6,274,597; 6,583,158; 6,589,964; 6,599,919; 6,916,829, and 7,141,582 all incorporated herein by reference). In particular, administration of small molecule derivatives of glucose and galactose, which are specific, selective competitive inhibitors for several target lysosomal enzymes, effectively increased the stability of the enzymes in cells *in vitro* and, thus, increased trafficking of the enzymes to the lysosome. Thus, by increasing the amount of enzyme in the lysosome, hydrolysis of the enzyme substrates is expected to increase. The original theory behind this strategy was as follows: since the mutant enzyme protein is unstable in the ER (Ishii et al., Biochem. Biophys. Res. Comm. 1996; 220: 812-815), the enzyme protein is retarded in the normal transport pathway (ER→Golgi apparatus→endosomes→lysosome) and prematurely degraded. Therefore, a compound which binds to and increases the stability of a mutant enzyme, may serve as a "chaperone" for the enzyme and increase the amount that can exit the ER and move to the lysosomes. In addition, because the folding and trafficking of some wild-type proteins is incomplete, with up to 70% of some wild-type proteins being degraded in some instances prior to reaching their final cellular location, the chaperones can be used to stabilize wild-type enzymes and increase the amount of enzyme which can exit the ER and be trafficked to lysosomes.

In one or more embodiments, the pharmacological chaperone comprises migalastat or a salt thereof. The compound migalastat, also known as 1-deoxygalactonojirimycin (1-DGJ) or (2R,3S,4R,5S)-2-(hydroxymethyl) piperdine-3,4,5-triol is a compound having the following chemical formula:

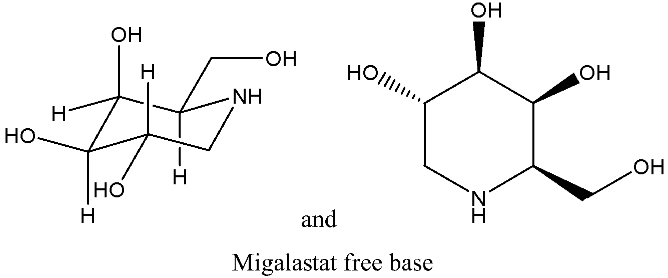

As discussed herein, pharmaceutically acceptable salts of migalastat may also be used in the present invention. When a salt of migalastat is used, the dosage of the salt will be adjusted so that the dose of migalastat received by the patient is equivalent to the amount which would have been received had the migalastat free base been used. One example of a pharmaceutically acceptable salt of migalastat is migalastat HCl:

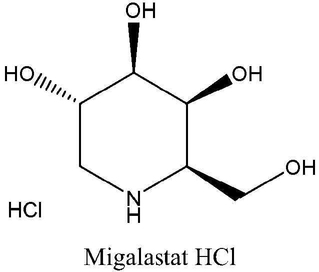

Migalastat is a low molecular weight iminosugar and is an analogue of the terminal galactose of GL-3. *In vitro* and *in vivo* pharmacologic studies have demonstrated that migalastat acts as a pharmacological chaperone, selectively and reversibly binding, with high affinity, to the active site of wild-type α-Gal A and specific mutant forms of α-Gal A, the genotypes of which are referred to as HEK assay amenable mutations. Migalastat binding stabilizes these mutant forms of α-Gal A in the endoplasmic reticulum facilitating their proper trafficking to lysosomes where dissociation of migalastat allows α-Gal A to reduce the level of GL-3 and other substrates. Approximately 30-50% of patients with Fabry disease have HEK assay amenable mutations; the majority of which are associated with the classic phenotype of the disease.

HEK assay amenable mutations include at least those mutations listed in a pharmacological reference table (*e.g.,* the ones recited in the U.S. or International Product labels for a migalastat product such as GALAFOLD^{®}). As used herein, "pharmacological reference table" refers to any publicly accessible written or electronic record, included in either the product label within the packaging of a migalastat product (e.g., GALAFOLD^{®}) or in a website accessible by health care providers, that conveys whether a particular mutation or variant is responsive to migalastat (*e.g.*, GALAFOLD^{®}) PC therapy, and is not necessarily limited to written records presented in tabular form. In one embodiment of the present invention, a "pharmacological reference table" thus refers to any depository of information that includes one or more amenable mutations or variants. An exemplary pharmacological reference table for HEK assay amenable mutations can be found in the summary of product characteristics and/or prescribing information for GALAFOLD^{®} in various countries in which GALAFOLD^{®} is approved for use, or at a website such as www.galafoldamenabilitytable.com or www.fabrygenevariantsearch.com, each of which is hereby incorporated by reference in its entirety.

Although the vast majority of a-GAL mutations are missense mutations, with most being outside the catalytic site, it difficult to predict which mutations result in an unstable enzyme that could be "rescued" by a pharmacological chaperone (PC) which stabilizes the enzyme, and which ones cannot be stabilized using a PC.

An exemplary pharmacological reference table for HEK assay amenable mutations is provided in Table 1 below. In one or more embodiments, if a double mutation is present on the same chromosome (males and females), that patient is considered HEK assay amenable if the double mutation is present in one entry in Table 1 (e.g., D55V/Q57L). In some embodiments, if a double mutation is present on different chromosomes (only in females) that patient is considered HEK assay amenable if either one of the individual mutations is present in Table 1.

**Table 1. HEK Assay Amenable Mutations**

| **Nucleotide change** | **Nucleotide change** | **Protein sequence change** |
|---|---|---|
| c.7C>G | c.C7G | L3V |
| c.8T>C | c.T8C | L3P |
| c.[11G>T; 620A>C] | c.G11T/A620C | R4M/Y207S |
| c.13A>G | c.A13G | N5D |
| c.15C>G | c.C15G | N5K |
| c.16C>A | c.C16A | P6T |
| c.16C>T | c.C16T | P6S |
| c.17C>A | c.C17A | P6Q |
| c.17C>G | c.C17G | P6R |
| c.17C>T | c.C17T | P6L |
| c.19G>A | c.G19A | E7K |
| c.20A>T | c.A20T | E7V |
| c.21A>T | c.A21T | E7D |
| c.22C>A | c.C22A | L8I |
| c.23T>A | c.T23A | L8Q |
| c.23T>C | c.T23C | L8P |
| c.25C>T | c.C25T | H9Y |
| c.26A>G | c.A26G | H9R |
| c.26A>T | c.A26T | H9L |
| c.27T>A | c.T27A | H9Q |
| c.28C>A | c.C28A | L10M |
| c.28C>G | c.C28G | L10V |
| c.29T>A | c.T29A | L10Q |
| c.29T>C | c.T29C | L10P |
| c.29T>G | c.T29G | L10R |
| c.31G>A | c.G31A | G11S |
| c.31G>C | c.G31C | G11R |
| c.31G>T | c.G31T | G11C |
| c.32G>A | c.G32A | G11D |
| c.32G>T | c.G32T | G11V |
| c.34T>A | c.T34A | C12S |
| c.34T>C | c.T34C | C12R |
| c.34T>G | c.T34G | C12G |
| c.35G>A | c.G35A | C12Y |
| c.37G>A | c.G37A | A13T |
| c.37G>C | c.G37C | A13P |
| c.38C>A | c.C38A | A13E |
| c.38C>G | c.C38G | A13G |
| c.40C>G | c.C40G | L14V |
| c.40C>T | c.C40T | L14F |
| c.41T>A | c.T41A | L14H |
| c.43G>A | c.G43A | A15T |
| c.44C>G | c.C44G | A15G |
| c.49C>A | c.C49A | R17S |
| c.49C>G | c.C49G | R17G |
| c.49C>T | c.C49T | R17C |
| c.50G>A | c.G50A | R17H |
| c.50G>C | c.G50C | R17P |
| c.52T>A | c.T52A | F18I |
| c.53T>G | c.T53G | F18C |
| c.54C>G | c.C54G | F18L |
| c.58G>C | c.G58C | A20P |
| c.59C>A | c.C59A | A20D |
| c.59C>G | c.C59G | A20G |
| c.62T>A | c.T62A | L21H |
| c.64G>A | c.G64A | V22I |
| c.64G>C | c.G64C | V22L |
| c.64G>T | c.G64T | V22F |
| c.65T>C | c.T65C | V22A |
| c.65T>G | c.T65G | V22G |
| c.67T>A | c.T67A | S23T |
| c.67T>C | c.T67C | S23P |
| c.70T>C or c.70T>A | c.T70C or c.T70A | W24R |
| c.70T>G | c.T70G | W24G |
| c.71G>C | c.G71C | W24S |
| c.72G>C or c.72G>T | c.G72C or c.G72T | W24C |
| c.73G>C | c.G73C | D25H |
| c.77T>A | c.T77A | I26N |
| c.79C>A | c.C79A | P27T |
| c.79C>G | c.C79G | P27A |
| c.79C>T | c.C79T | P27S |
| c.80C>T | c.C80T | P27L |
| c.82G>C | c.G82C | G28R |
| c.82G>T | c.G82T | G28W |
| c.83G>A | c.G83A | G28E |
| c.85G>C | c.G85C | A29P |
| c.86C>A | c.C86A | A29D |
| c.86C>G | c.C86G | A29G |
| c.86C>T | c.C86T | A29V |
| c.88A>G | c.A88G | R30G |
| c.94C>A | c.C94A | L32M |
| c.94C>G | c.C94G | L32V |
| c.95T>A | c.T95A | L32Q |
| c.95T>C | c.T95C | L32P |
| c.95T>G | c.T95G | L32R |
| c.97G>C | c.G97C | D33H |
| c.97G>T | c.G97T | D33Y |
| c.98A>C | c.A98C | D33A |
| c.98A>G | c.A98G | D33G |
| c.98A>T | c.A98T | D33V |
| c.99C>G | c.C99G | D33E |
| c.100A>C | c.A100C | N34H |
| c.100A>G | c.A100G | N34D |
| c.101A>C | c.A101C | N34T |
| c.101A>G | c.A101G | N34S |
| c.102T>G or c.102T>A | c.T102G or c.T102A | N34K |
| c.103G>C or c.103G>A | c.G103C or c.G103A | G35R |
| c.104G>A | c.G104A | G35E |
| c.104G>C | c.G104C | G35A |
| c.104G>T | c.G104T | G35V |
| c.106T>A | c.T106A | L36M |
| c.106T>G | c.T106G | L36V |
| c.107T>C | c.T107C | L36S |
| c.107T>G | c.T107G | L36W |
| c.108G>C or c.108G>T | c.G108C or c.G108T | L36F |
| c.109G>A | c.G109A | A37T |
| c.109G>T | c.G109T | A37S |
| c.110C>A | c.C110A | A37E |
| c.110C>G | c.C110G | A37G |
| c.110C>T | c.C110T | A37V |
| c.112A>G | c.A112G | R38G |
| c.112A>T | c.A112T | R38W |
| c.113G>T | c.G113T | R38M |
| c.114G>C | c.G114C | R38S |
| c.115A>G | c.A115G | T39A |
| c.115A>T | c.A115T | T39S |
| c.116C>A | c.C116A | T39K |
| c.116C>G | c.C116G | T39R |
| c.116C>T | c.C116T | T39M |
| c.121A>G | c.A121G | T41A |
| c.122C>A | c.C122A | T41N |
| c.122C>G | c.C122G | T41S |
| c.122C>T | c.C122T | T41I |
| c.124A>C or c.124A>T | c.A124C or c.A124T | M42L |
| c.124A>G | c.A124G | M42V |
| c.125T>A | c.T125A | M42K |
| c.125T>C | c.T125C | M42T |
| c.125T>G | c.T125G | M42R |
| c.126G>A or c.126G>C or c.126G>T | c.G126A or c.G126C or c.G126T | M42I |
| c.128G>C | c.G128C | G43A |
| c.133C>A | c.C133A | L45M |
| c.133C>G | c.C133G | L45V |
| c.136C>A | c.C136A | H46N |
| c.136C>G | c.C136G | H46D |
| c.137A>C | c.A137C | H46P |
| c.138C>G | c.C138G | H46Q |
| c.142G>C | c.G142C | E48Q |
| c.143A>C | c.A143C | E48A |
| c.149T>A | c.T149A | F50Y |
| c.151A>G | c.A151G | M51V |
| c.152T>A | c.T152A | M51K |
| c.152T>C | c.T152C | M51T |
| c.152T>G | c.T152G | M51R |
| c.153G>A or c.153G>T or c.153G>C | c.G153A or c.G153T or c.G153C | M51I |
| c.157A>C | c.A157C | N53H |
| c.[157A>C; 158A>T] | c.A157C/A158T | N53L |
| c.157A>G | c.A157G | N53D |
| c.157A>T | c.A157T | N53Y |
| c.158A>C | c.A158C | N53T |
| c.158A>G | c.A158G | N53S |
| c.158A>T | c.A158T | N53I |
| c.159C>G or c.159C>A | c.C159G or c.C159A | N53K |
| c.160C>G | c.C160G | L54V |
| c.160C>T | c.C160T | L54F |
| c.161T>A | c.T161A | L54H |
| c.161T>C | c.T161C | L54P |
| c.161T>G | c.T161G | L54R |
| c.163G>C | c.G163C | D55H |
| c.163G>T | c.G163T | D55Y |
| c.164A>C | c.A164C | D55A |
| c.164A>G | c.A164G | D55G |
| c.164A>T | c.A164T | D55V |
| c.[164A>T; 170A>T] | c.A164T/A170T | D55V/Q57L |
| c.165C>G | c.C165G | D55E |
| c.167G>A | c.G167A | C56Y |
| c.167G>T | c.G167T | C56F |
| c.168C>G | c.C168G | C56W |
| c.170A>G | c.A170G | Q57R |
| c.170A>T | c.A170T | Q57L |
| c.172G>A | c.G172A | E58K |
| c.175G>A | c.G175A | E59K |
| c.175G>C | c.G175C | E59Q |
| c.176A>C | c.A176C | E59A |
| c.176A>G | c.A176G | E59G |
| c.176A>T | c.A176T | E59V |
| c.177G>C | c.G177C | E59D |
| c.178C>A | c.C178A | P60T |
| c.178C>G | c.C178G | P60A |
| c.178C>T | c.C178T | P60S |
| c.179C>A | c.C179A | P60Q |
| c.179C>G | c.C179G | P60R |
| c.179C>T | c.C179T | P60L |
| c.182A>T | c.A182T | D61V |
| c.183T>A | c.T183A | D61E |
| c.184 185insTAG | c.184 185insTAG | S62delinsLA |
| c.184T>C | c.T184C | S62P |
| c.184T>G | c.T184G | S62A |
| c.185C>A | c.C185A | S62Y |
| c.185C>G | c.C185G | S62C |
| c.185C>T | c.C185T | S62F |
| c.190A>C | c.A190C | I64L |
| c.190A>G | c.A190G | I64V |
| c.193A>G | c.A193G | S65G |
| c.193A>T | c.A193T | S65C |
| c.195T>A | c.T195A | S65R |
| c.196G>A | c.G196A | E66K |
| c.197A>G | c.A197G | E66G |
| c.197A>T | c.A197T | E66V |
| c.198G>C | c.G198C | E66D |
| c.199A>C | c.A199C | K67Q |
| c.199A>G | c.A199G | K67E |
| c.200A>C | c.A200C | K67T |
| c.200A>T | c.A200T | K67M |
| c.201G>C | c.G201C | K67N |
| c.202C>A | c.C202A | L68I |
| c.205T>A | c.T205A | F69I |
| c.206T>A | c.T206A | F69Y |
| c.207C>A or c.207C>G | c.C207A or c.C207G | F69L |
| c.208A>T | c.A208T | M70L |
| c.209T>A | c.T209A | M70K |
| c.209T>G | c.T209G | M70R |
| c.210G>C | c.G210C | M70I |
| c.211G>C | c.G211C | E71Q |
| c.212A>C | c.A212C | E71A |
| c.212A>G | c.A212G | E71G |
| c.212A>T | c.A212T | E71V |
| c.213G>C | c.G213C | E71D |
| c.214A>G | c.A214G | M72V |
| c.214A>T | c.A214T | M72L |
| c.215T>C | c.T215C | M72T |
| c.216G>A or c.216G>T or c.216G>C | c.G216A or c.G216T or c.G216C | M72I |
| c.217G>A | c.G217A | A73T |
| c.217G>T | c.G217T | A73S |
| c.218C>T | c.C218T | A73V |
| c.220G>A | c.G220A | E74K |
| c.221A>G | c.A221G | E74G |
| c.221A>T | c.A221T | E74V |
| c.222G>C | c.G222C | E74D |
| c.223C>T | c.C223T | L75F |
| c.224T>C | c.T224C | L75P |
| c.226A>G | c.A226G | M76V |
| c.227T>C | c.T227C | M76T |
| c.229G>A | c.G229A | V77I |
| c.229G>C | c.G229C | V77L |
| c.232T>C | c.T232C | S78P |
| c.233C>T | c.C233T | S78L |
| c.235G>A | c.G235A | E79K |
| c.235G>C | c.G235C | E79Q |
| c.236A>C | c.A236C | E79A |
| c.236A>G | c.A236G | E79G |
| c.236A>T | c.A236T | E79V |
| c.237A>T | c.A237T | E79D |
| c.238G>A | c.G238A | G80S |
| c.238G>T | c.G238T | G80C |
| c.239G>A | c.G239A | G80D |
| c.239G>C | c.G239C | G80A |
| c.239G>T | c.G239T | G80V |
| c.242G>T | c.G242T | W81L |
| c.244A>G | c.A244G | K82E |
| c.245A>C | c.A245C | K82T |
| c.245A>G | c.A245G | K82R |
| c.245A>T | c.A245T | K82M |
| c.246G>C | c.G246C | K82N |
| c.247G>A | c.G247A | D83N |
| c.248A>C | c.A248C | D83A |
| c.248A>G | c.A248G | D83G |
| c.248A>T | c.A248T | D83V |
| c.249T>A | c.T249A | D83E |
| c.250G>A | c.G250A | A84T |
| c.250G>C | c.G250C | A84P |
| c.250G>T | c.G250T | A84S |
| c.251C>A | c.C251A | A84E |
| c.251C>G | c.C251G | A84G |
| c.251C>T | c.C251T | A84V |
| c.253G>A | c.G253A | G85S |
| c.[253G>A; 254G>A] | c.G253A/G254A | G85N |
| c.[253G>A; 254G>T; 255T>G] | c.G253A/G254T/T255G | G85M |
| c.253G>C | c.G253C | G85R |
| c.253G>T | c.G253T | G85C |
| c.254G>A | c.G254A | G85D |
| c.254G>C | c.G254C | G85A |
| c.257A>T | c.A257T | Y86F |
| c.260A>G | c.A260G | E87G |
| c.261G>C or c.261G>T | c.G261C or c.G261T | E87D |
| c.262T>A | c.T262A | Y88N |
| c.262T>C | c.T262C | Y88H |
| c.263A>C | c.A263C | Y88S |
| c.263A>G | c.A263G | Y88C |
| c.265C>G | c.C265G | L89V |
| c.265C>T | c.C265T | L89F |
| c.271A>C | c.A271C | I91L |
| c.271A>T | c.A271T | I91F |
| c.272T>C | c.T272C | I91T |
| c.272T>G | c.T272G | I91S |
| c.273T>G | c.T273G | I91M |
| c.286A>G | c.A286G | M96V |
| c.286A>T | c.A286T | M96L |
| c.287T>C | c.T287C | M96T |
| c.288G>A or c.288G>T or c.288G>C | c.G288A or c.G288T or c.G288C | M96I |
| c.289G>A | c.G289A | A97T |
| c.289G>C | c.G289C | A97P |
| c.289G>T | c.G289T | A97S |
| c.290C>A | c.C290A | A97D |
| c.290C>T | c.C290T | A97V |
| c.293C>A | c.C293A | P98H |
| c.293C>G | c.C293G | P98R |
| c.293C>T | c.C293T | P98L |
| c.295C>G | c.C295G | Q99E |
| c.296A>C | c.A296C | Q99P |
| c.296A>G | c.A296G | Q99R |
| c.296A>T | c.A296T | Q99L |
| c.301G>C | c.G301C | D101H |
| c.302A>C | c.A302C | D101A |
| c.302A>G | c.A302G | D101G |
| c.302A>T | c.A302T | D101V |
| c.303T>A | c.T303A | D101E |
| c.304T>A | c.T304A | S102T |
| c.304T>C | c.T304C | S102P |
| c.304T>G | c.T304G | S102A |
| c.305C>T | c.C305T | S102L |
| c.310G>A | c.G310A | G104S |
| c.311G>A | c.G311A | G104D |
| c.311G>C | c.G311C | G104A |
| c.311G>T | c.G311T | G104V |
| c.313A>G | c.A313G | R105G |
| c.314G>A | c.G314A | R105K |
| c.314G>C | c.G314C | R105T |
| c.314G>T | c.G314T | R105I |
| c.316C>A | c.C316A | L106I |
| c.316C>G | c.C316G | L106V |
| c.316C>T | c.C316T | L106F |
| c.317T>A | c.T317A | L106H |
| c.317T>C | c.T317C | L106P |
| c.319C>A | c.C319A | Q107K |
| c.319C>G | c.C319G | Q107E |
| c.320A>G | c.A320G | Q107R |
| c.321G>C | c.G321C | Q107H |
| c.322G>A | c.G322A | A108T |
| c.323C>A | c.C323A | A108E |
| c.323C>T | c.C323T | A108V |
| c.325G>A | c.G325A | D109N |
| c.325G>C | c.G325C | D109H |
| c.325G>T | c.G325T | D109Y |
| c.326A>C | c.A326C | D109A |
| c.326A>G | c.A326G | D109G |
| c.327C>G | c.C327G | D109E |
| c.328C>A | c.C328A | P110T |
| c.334C>G | c.C334G | R112G |
| c.335G>A | c.G335A | R112H |
| c.335G>T | c.G335T | R112L |
| c.337T>A | c.T337A | F113I |
| c.337T>C or c.339T>A or c.339T>G | c.T337C or c.T339A or c.T339G | F113L |
| c.337T>G | c.T337G | F113V |
| c.338T>A | c.T338A | F113Y |
| c.341C>T | c.C341T | P114L |
| c.343C>A | c.C343A | H115N |
| c.343C>G | c.C343G | H115D |
| c.346G>C | c.G346C | G116R |
| c.350T>C | c.T350C | I117T |
| c.351T>G | c.T351G | I117M |
| c.352C>T | c.C352T | R118C |
| c.361G>A | c.G361A | A121T |
| c.362C>T | c.C362T | A121V |
| c.367T>A | c.T367A | Y123N |
| c.367T>G | c.T367G | Y123D |
| c.368A>C | c.A368C | Y123S |
| c.368A>G | c.A368G | Y123C |
| c.368A>T | c.A368T | Y123F |
| c.370G>A | c.G370A | V124I |
| c.371T>G | c.T371G | V124G |
| c.373C>A | c.C373A | H125N |
| c.373C>G | c.C373G | H125D |
| c.373C>T | c.C373T | H125Y |
| c.374A>G | c.A374G | H125R |
| c.374A>T | c.A374T | H125L |
| c.376A>G | c.A376G | S126G |
| c.376A>T | c.A376T | S126C |
| c.377G>T | c.G377T | S1261 |
| c.379A>G | c.A379G | K127E |
| c.383G>A | c.G383A | G128E |
| c.383G>C | c.G383C | G128A |
| c.385C>G | c.C385G | L129V |
| c.388A>C | c.A388C | K130Q |
| c.389A>T | c.A389T | K130M |
| c.390G>C | c.G390C | K130N |
| c.391C>G | c.C391G | L131V |
| c.397A>C | c.A397C | I133L |
| c.397A>G | c.A397G | I133V |
| c.397A>T | c.A397T | I133F |
| c.398T>C | c.T398C | I133T |
| c.399T>G | c.T399G | I133M |
| c.[399T>G; 434T>C] | c.T399G/T434C | I133M/F145S |
| c.403G>A | c.G403A | A135T |
| c.403G>T | c.G403T | A135S |
| c.404C>A | c.C404A | A135E |
| c.404C>G | c.C404G | A135G |
| c.404C>T | c.C404T | A135V |
| c.406G>A | c.G406A | D136N |
| c.407A>C | c.A407C | D136A |
| c.407A>T | c.A407T | D136V |
| c.408T>A or c.408T>G | c.T408A or c.T408G | D136E |
| c.409G>A | c.G409A | V137I |
| c.409G>C | c.G409C | V137L |
| c.410T>A | c.T410A | V137D |
| c.410T>C | c.T410C | V137A |
| c.410T>G | c.T410G | V137G |
| c.413G>C | c.G413C | G138A |
| c.415A>C | c.A415C | N139H |
| c.415A>T | c.A415T | N139Y |
| c.416A>G | c.A416G | N139S |
| c.416A>T | c.A416T | N139I |
| c.417T>A | c.T417A | N139K |
| c.418A>C | c.A418C | K140Q |
| c.418A>G | c.A418G | K140E |
| c.419A>C | c.A419C | K140T |
| c.419A>G | c.A419G | K140R |
| c.419A>T | c.A419T | K140I |
| c.420A>T | c.A420T | K140N |
| c.421A>T | c.A421T | T141S |
| c.427G>A | c.G427A | A143T |
| c.428C>A | c.C428A | A143E |
| c.428C>G | c.C428G | A143G |
| c.428C>T | c.C428T | A143V |
| c.430G>A | c.G430A | G144S |
| c.430G>C | c.G430C | G144R |
| c.430G>T | c.G430T | G144C |
| c.431G>A | c.G431A | G144D |
| c.431G>C | c.G431C | G144A |
| c.431G>T | c.G431T | G144V |
| c.433T>G | c.T433G | F145V |
| c.434T>A | c.T434A | F145Y |
| c.434T>C | c.T434C | F145S |
| c.434T>G | c.T434G | F145C |
| c.435C>G | c.C435G | F145L |
| c.436C>A | c.C436A | P146T |
| c.436C>G | c.C436G | P146A |
| c.436C>T | c.C436T | P146S |
| c.437C>A | c.C437A | P146H |
| c.437C>G | c.C437G | P146R |
| c.437C>T | c.C437T | P146L |
| c.440G>C | c.G440C | G147A |
| c.442A>G | c.A442G | S148G |
| c.442A>T | c.A442T | S148C |
| c.443G>C | c.G443C | S148T |
| c.446T>G | c.T446G | F149C |
| c.449G>A | c.G449A | G150E |
| c.449G>T | c.G449T | G150V |
| c.451T>G | c.T451G | Y151D |
| c.452A>C | c.A452C | Y151S |
| c.452A>G | c.A452G | Y151C |
| c.454T>A | c.T454A | Y152N |
| c.454T>C | c.T454C | Y152H |
| c.454T>G | c.T454G | Y152D |
| c.455A>C | c.A455C | Y152S |
| c.455A>G | c.A455G | Y152C |
| c.455A>T | c.A455T | Y152F |
| c.457G>A | c.G457A | D153N |
| c.457G>C | c.G457C | D153H |
| c.457G>T | c.G457T | D153Y |
| c.458A>C | c.A458C | D153A |
| c.458A>T | c.A458T | D153V |
| c.465T>A or c.465T>G | c.T465A or c.T465G | D155E |
| c.466G>A | c.G466A | A156T |
| c.466G>T | c.G466T | A156S |
| c.467C>G | c.C467G | A156G |
| c.467C>T | c.C467T | A156V |
| c.469C>A | c.C469A | Q157K |
| c.469C>G | c.C469G | Q157E |
| c.470A>C | c.A470C | Q157P |
| c.470A>T | c.A470T | Q157L |
| c.471G>C or c.471G>T | c.G471C or c.G471T | Q157H |
| c.472A>G | c.A472G | T158A |
| c.472A>T | c.A472T | T158S |
| c.473C>A | c.C473A | T158N |
| c.473C>T | c.C473T | T158I |
| c.475T>A | c.T475A | F159I |
| c.475T>G | c.T475G | F159V |
| c.476T>A | c.T476A | F159Y |
| c.476T>G | c.T476G | F159C |
| c.477T>A | c.T477A | F159L |
| c.478G>A | c.G478A | A160T |
| c.478G>T | c.G478T | A160S |
| c.479C>A | c.C479A | A160D |
| c.479C>G | c.C479G | A160G |
| c.479C>T | c.C479T | A160V |
| c.481G>A | c.G481A | D161N |
| c.481G>C | c.G481C | D161H |
| c.481G>T | c.G481T | D161Y |
| c.482A>T | c.A482T | D161V |
| c.484T>G | c.T484G | W162G |
| c.485G>C | c.G485C | W162S |
| c.490G>A | c.G490A | V164I |
| c.490G>T | c.G490T | V164L |
| c.491T>C | c.T491C | V164A |
| c.493G>A | c.G493A | D165N |
| c.493G>C | c.G493C | D165H |
| c.494A>C | c.A494C | D165A |
| c.494A>G | c.A494G | D165G |
| c.495T>A | c.T495A | D165E |
| c.496 497delinsTC | c.496 497delinsTC | L166S |
| c.496C>A | c.C496A | L166M |
| c.496C>G | c.C496G | L166V |
| c.[496C>G; 497T>G] | c.C496G/T497G | L166G |
| c.497T>A | c.T497A | L166Q |
| c.499C>A | c.C499A | L167I |
| c.499C>G | c.C499G | L167V |
| c.505T>A | c.T505A | F169I |
| c.505T>G | c.T505G | F169V |
| c.506T>A | c.T506A | F169Y |
| c.506T>C | c.T506C | F169S |
| c.506T>G | c.T506G | F169C |
| c.507T>A | c.T507A | F169L |
| c.511G>A | c.G511A | G171S |
| c.512G>C | c.G512C | G171A |
| c.512G>T | c.G512T | G171V |
| c.517T>C | c.T517C | Y173H |
| c.518A>C | c.A518C | Y173S |
| c.518A>G | c.A518G | Y173C |
| c.518A>T | c.A518T | Y173F |
| c.520T>C | c.T520C | C174R |
| c.520T>G | c.T520G | C174G |
| c.523G>C | c.G523C | D175H |
| c.523G>T | c.G523T | D175Y |
| c.524A>G | c.A524G | D175G |
| c.524A>T | c.A524T | D175V |
| c.525C>G or c.525C>A | c.C525G or c.C525A | D175E |
| c.526A>T | c.A526T | S176C |
| c.528T>A | c.T528A | S176R |
| c.529T>A | c.T529A | L177M |
| c.529T>G | c.T529G | L177V |
| c.530T>C | c.T530C | L177S |
| c.530T>G | c.T530G | L177W |
| c.531G>C | c.G531C | L177F |
| c.532G>A | c.G532A | E178K |
| c.532G>C | c.G532C | E178Q |
| c.533A>C | c.A533C | E178A |
| c.533A>G | c.A533G | E178G |
| c.538T>A | c.T538A | L180M |
| c.538T>G | c.T538G | L180V |
| c.539T>C | c.T539C | L180S |
| c.539T>G | c.T539G | L180W |
| c.540G>C or c.540G>T | c.G540C or c.G540T | L180F |
| c.541G>A | c.G541A | A181T |
| c.541G>C | c.G541C | A181P |
| c.542C>T | c.C542T | A181V |
| c.544G>T | c.G544T | D182Y |
| c.545A>C | c.A545C | D182A |
| c.545A>G | c.A545G | D182G |
| c.545A>T | c.A545T | D182V |
| c.546T>A | c.T546A | D182E |
| c.548G>A | c.G548A | G183D |
| c.548G>C | c.G548C | G183A |
| c.550T>A | c.T550A | Y184N |
| c.550T>C | c.T550C | Y184H |
| c.551A>C | c.A551C | Y184S |
| c.551A>G | c.A551G | Y184C |
| c.551A>T | c.A551T | Y184F |
| c.553A>C | c.A553C | K185Q |
| c.553A>G | c.A553G | K185E |
| c.554A>C | c.A554C | K185T |
| c.554A>T | c.A554T | K185M |
| c.555G>C | c.G555C | K185N |
| c.556C>A | c.C556A | H186N |
| c.556C>G | c.C556G | H186D |
| c.556C>T | c.C556T | H186Y |
| c.557A>T | c.A557T | H186L |
| c.558C>G | c.C558G | H186Q |
| c.559 564dup | c.559 564dup | p.M187_S188dup |
| c.559A>T | c.A559T | M187L |
| c.559A>G | c.A559G | M187V |
| c.560T>C | c.T560C | M187T |
| c.561G>T or c.561G>A or c.561G>C | c.G561T or c.G561A or c.G561C | M187I |
| c.562T>A | c.T562A | S188T |
| c.562T>C | c.T562C | S188P |
| c.562T>G | c.T562G | S188A |
| c.563C>A | c.C563A | S188Y |
| c.563C>G | c.C563G | S188C |
| c.563C>T | c.C563T | S188F |
| c.565T>G | c.T565G | L189V |
| c.566T>C | c.T566C | L189S |
| c.567G>C or c.567G>T | c.G567C or c.G567T | L189F |
| c.568G>A | c.G568A | A190T |
| c.568G>T | c.G568T | A190S |
| c.569C>A | c.C569A | A190D |
| c.569C>G | c.C569G | A190G |
| c.569C>T | c.C569T | A190V |
| c.571C>A | c.C571A | L191M |
| c.571C>G | c.C571G | L191V |
| c.572T>A | c.T572A | L191Q |
| c.574A>C | c.A574C | N192H |
| c.574A>G | c.A574G | N192D |
| c.575A>C | c.A575C | N192T |
| c.575A>G | c.A575G | N192S |
| c.576T>A | c.T576A | N192K |
| c.577A>G | c.A577G | R193G |
| c.577A>T | c.A577T | R193W |
| c.578G>C | c.G578C | R193T |
| c.578G>T | c.G578T | R193M |
| c.580A>C | c.A580C | T194P |
| c.580A>G | c.A580G | T194A |
| c.580A>T or c.581C>G | c.A580T or c.C581G | T194S |
| c.581C>A | c.C581A | T194N |
| c.581C>T | c.C581T | T194I |
| c.583G>A | c.G583A | G195S |
| c.583G>C | c.G583C | G195R |
| c.583G>T | c.G583T | G195C |
| c.584G>T | c.G584T | G195V |
| c.586A>G | c.A586G | R196G |
| c.587G>A | c.G587A | R196K |
| c.587G>C | c.G587C | R196T |
| c.587G>T | c.G587T | R196I |
| c.589A>G | c.A589G | S197G |
| c.589A>T | c.A589T | S197C |
| c.590G>A | c.G590A | S197N |
| c.590G>C | c.G590C | S197T |
| c.590G>T | c.G590T | S197I |
| c.593T>C | c.T593C | I198T |
| c.593T>G | c.T593G | I198S |
| c.594T>G | c.T594G | I198M |
| c.595G>A | c.G595A | V199M |
| c.595G>C | c.G595C | V199L |
| c.596T>A | c.T596A | V199E |
| c.596T>C | c.T596C | V199A |
| c.596T>G | c.T596G | V199G |
| c.598T>A | c.T598A | Y200N |
| c.599A>C | c.A599C | Y200S |
| c.599A>G | c.A599G | Y200C |
| c.601T>A | c.T601A | S201T |
| c.601T>G | c.T601G | S201A |
| c.602C>A | c.C602A | S201Y |
| c.602C>G | c.C602G | S201C |
| c.602C>T | c.C602T | S201F |
| c.607G>C | c.G607C | E203Q |
| c.608A>C | c.A608C | E203A |
| c.608A>G | c.A608G | E203G |
| c.608A>T | c.A608T | E203V |
| c.609G>C or c.609G>T | c.G609C or c.G609T | E203D |
| c.610T>G | c.T610G | W204G |
| c.611G>C | c.G611C | W204S |
| c.611G>T | c.G611T | W204L |
| c.613C>A | c.C613A | P205T |
| c.613C>T | c.C613T | P205S |
| c.614C>T | c.C614T | P205L |
| c.616C>A | c.C616A | L206I |
| c.616C>G | c.C616G | L206V |
| c.616C>T | c.C616T | L206F |
| c.617T>A | c.T617A | L206H |
| c.617T>G | c.T617G | L206R |
| c.619T>C | c.T619C | Y207H |
| c.620A>C | c.A620C | Y207S |
| c.620A>T | c.A620T | Y207F |
| c.623T>A | c.T623A | M208K |
| c.623T>G | c.T623G | M208R |
| c.625T>A | c.T625A | W209R |
| c.625T>G | c.T625G | W209G |
| c.627G>C | c.G627C | W209C |
| c.628C>A | c.C628A | P210T |
| c.628C>T | c.C628T | P210S |
| c.629C>A | c.C629A | P210H |
| c.629C>T | c.C629T | P210L |
| c.631T>C | c.T631C | F211L |
| c.631T>G | c.T631G | F211V |
| c.632T>A | c.T632A | F211Y |
| c.632T>C | c.T632C | F211S |
| c.632T>G | c.T632G | F211C |
| c.635A>C | c.A635C | Q212P |
| c.636A>T | c.A636T | Q212H |
| c.637A>C | c.A637C | K213Q |
| c.637A>G | c.A637G | K213E |
| c.638A>G | c.A638G | K213R |
| c.638A>T | c.A638T | K213M |
| c.640C>A | c.C640A | P214T |
| c.640C>G | c.C640G | P214A |
| c.640C>T | c.C640T | P214S |
| c.641C>A | c.C641A | P214H |
| c.641C>G | c.C641G | P214R |
| c.641C>T | c.C641T | P214L |
| c.643A>C | c.A643C | N215H |
| c.643A>G | c.A643G | N215D |
| c.643A>T | c.A643T | N215Y |
| c.644A>C | c.A644C | N215T |
| c.644A>G | c.A644G | N215S |
| c.[644A>G; 937G>T] | c.A644G/G937T | N215S/D313Y |
| c.644A>T | c.A644T | N215I |
| c.645T>A | c.T645A | N215K |
| c.646T>A | c.T646A | Y216N |
| c.646T>C | c.T646C | Y216H |
| c.646T>G | c.T646G | Y216D |
| c.647A>C | c.A647C | Y216S |
| c.647A>G | c.A647G | Y216C |
| c.647A>T | c.A647T | Y216F |
| c.649A>C | c.A649C | T217P |
| c.649A>G | c.A649G | T217A |
| c.649A>T | c.A649T | T217S |
| c.650C>A | c.C650A | T217K |
| c.650C>G | c.C650G | T217R |
| c.650C>T | c.C650T | T217I |
| c.652G>A | c.G652A | E218K |
| c.652G>C | c.G652C | E218Q |
| c.653A>C | c.A653C | E218A |
| c.653A>G | c.A653G | E218G |
| c.653A>T | c.A653T | E218V |
| c.654A>T | c.A654T | E218D |
| c.655A>C | c.A655C | 1219L |
| c.655A>T | c.A655T | I219F |
| c.656T>A | c.T656A | I219N |
| c.656T>C | c.T656C | I219T |
| c.656T>G | c.T656G | I219S |
| c.657C>G | c.C657G | I219M |
| c.659G>A | c.G659A | R220Q |
| c.659G>C | c.G659C | R220P |
| c.659G>T | c.G659T | R220L |
| c.661C>A | c.C661A | Q221K |
| c.661C>G | c.C661G | Q221E |
| c.662A>C | c.A662C | Q221P |
| c.662A>G | c.A662G | Q221R |
| c.662A>T | c.A662T | Q221L |
| c.663G>C | c.G663C | Q221H |
| c.664T>A | c.T664A | Y222N |
| c.664T>C | c.T664C | Y222H |
| c.664T>G | c.T664G | Y222D |
| c.665A>C | c.A665C | Y222S |
| c.665A>G | c.A665G | Y222C |
| c.670A>C | c.A670C | N224H |
| c.671A>C | c.A671C | N224T |
| c.671A>G | c.A671G | N224S |
| c.673C>G | c.C673G | H225D |
| c.679C>G | c.C679G | R227G |
| c.682A>C | c.A682C | N228H |
| c.682A>G | c.A682G | N228D |
| c.683A>C | c.A683C | N228T |
| c.683A>G | c.A683G | N228S |
| c.683A>T | c.A683T | N228I |
| c.685T>A | c.T685A | F229I |
| c.686T>A | c.T686A | F229Y |
| c.686T>C | c.T686C | F229S |
| c.687T>A or c.687T>G | c.T687A or c.T687G | F229L |
| c.688G>C | c.G688C | A230P |
| c.689C>A | c.C689A | A230D |
| c.689C>G | c.C689G | A230G |
| c.689C>T | c.C689T | A230V |
| c.694A>C | c.A694C | I232L |
| c.694A>G | c.A694G | I232V |
| c.695T>C | c.T695C | I232T |
| c.696T>G | c.T696G | I232M |
| c.698A>C | c.A698C | D233A |
| c.698A>G | c.A698G | D233G |
| c.698A>T | c.A698T | D233V |
| c.699T>A | c.T699A | D233E |
| c.703T>A | c.T703A | S235T |
| c.703T>G | c.T703G | S235A |
| c.710A>T | c.A710T | K237I |
| c.712A>G | c.A712G | S238G |
| c.712A>T | c.A712T | S238C |
| c.713G>A | c.G713A | S238N |
| c.713G>C | c.G713C | S238T |
| c.713G>T | c.G713T | S238I |
| c.715A>T | c.A715T | I239L |
| c.716T>C | c.T716C | I239T |
| c.717A>G | c.A717G | I239M |
| c.718A>G | c.A718G | K240E |
| c.719A>G | c.A719G | K240R |
| c.719A>T | c.A719T | K240M |
| c.720G>C or c.720G>T | c.G720C or c.G720T | K240N |
| c.721A>T | c.A721T | S241C |
| c.722G>C | c.G722C | S241T |
| c.722G>T | c.G722T | S241I |
| c.724A>C | c.A724C | I242L |
| c.724A>G | c.A724G | I242V |
| c.724A>T | c.A724T | I242F |
| c.725T>A | c.T725A | I242N |
| c.725T>C | c.T725C | I242T |
| c.725T>G | c.T725G | I242S |
| c.726C>G | c.C726G | I242M |
| c.727T>A | c.T727A | L243M |
| c.727T>G | c.T727G | L243V |
| c.728T>C | c.T728C | L243S |
| c.728T>G | c.T728G | L243W |
| c.729G>C or c.729G>T | c.G729C or c.G729T | L243F |
| c.730G>A | c.G730A | D244N |
| c.730G>C | c.G730C | D244H |
| c.730G>T | c.G730T | D244Y |
| c.731A>C | c.A731C | D244A |
| c.731A>G | c.A731G | D244G |
| c.731A>T | c.A731T | D244V |
| c.732C>G | c.C732G | D244E |
| c.733T>G | c.T733G | W245G |
| c.735G>C | c.G735C | W245C |
| c.736A>G | c.A736G | T246A |
| c.737C>A | c.C737A | T246K |
| c.737C>G | c.C737G | T246R |
| c.737C>T | c.C737T | T246I |
| c.739T>A | c.T739A | S247T |
| c.739T>G | c.T739G | S247A |
| c.740C>A | c.C740A | S247Y |
| c.740C>G | c.C740G | S247C |
| c.740C>T | c.C740T | S247F |
| c.742T>G | c.T742G | F248V |
| c.743T>A | c.T743A | F248Y |
| c.743T>G | c.T743G | F248C |
| c.744T>A | c.T744A | F248L |
| c.745A>C | c.A745C | N249H |
| c.745A>G | c.A745G | N249D |
| c.745A>T | c.A745T | N249Y |
| c.746A>C | c.A746C | N249T |
| c.746A>G | c.A746G | N249S |
| c.746A>T | c.A746T | N249I |
| c.747C>G or c.747C>A | c.C747G or c.C747A | N249K |
| c.748C>A | c.C748A | Q250K |
| c.748C>G | c.C748G | Q250E |
| c.749A>C | c.A749C | Q250P |
| c.749A>G | c.A749G | Q250R |
| c.749A>T | c.A749T | Q250L |
| c.750G>C | c.G750C | Q250H |
| c.751G>A | c.G751A | E251K |
| c.751G>C | c.G751C | E251Q |
| c.752A>G | c.A752G | E251G |
| c.752A>T | c.A752T | E251V |
| c.754A>G | c.A754G | R252G |
| c.757A>G | c.A757G | I253V |
| c.757A>T | c.A757T | I253F |
| c.758T>A | c.T758A | I253N |
| c.758T>C | c.T758C | I253T |
| c.758T>G | c.T758G | I253S |
| c.760-762de1GTT or c.761-763del | c.760_762delGTT or c.761 763del | p.V254de1 |
| c.760G>T | c.G760T | V254F |
| c.761T>A | c.T761A | V254D |
| c.761T>C | c.T761C | V254A |
| c.761T>G | c.T761G | V254G |
| c.763G>A | c.G763A | D255N |
| c.763G>C | c.G763C | D255H |
| c.763G>T | c.G763T | D255Y |
| c.764A>C | c.A764C | D255A |
| c.764A>T | c.A764T | D255V |
| c.765T>A | c.T765A | D255E |
| c.766G>C | c.G766C | V256L |
| c.767T>A | c.T767A | V256D |
| c.767T>G | c.T767G | V256G |
| c.769G>A | c.G769A | A257T |
| c.769G>C | c.G769C | A257P |
| c.769G>T | c.G769T | A257S |
| c.770C>G | c.C770G | A257G |
| c.770C>T | c.C770T | A257V |
| c.772G>C or c.772G>A | c.G772C or c.G772A | G258R |
| c.773G>A | c.G773A | G258E |
| c.773G>T | c.G773T | G258V |
| c.775C>A | c.C775A | P259T |
| c.775C>G | c.C775G | P259A |
| c.775C>T | c.C775T | P259S |
| c.776C>A | c.C776A | P259Q |
| c.776C>G | c.C776G | P259R |
| c.776C>T | c.C776T | P259L |
| c.778G>T | c.G778T | G260W |
| c.779G>A | c.G779A | G260E |
| c.779G>C | c.G779C | G260A |
| c.781G>A | c.G781A | G261S |
| c.781G>C | c.G781C | G261R |
| c.781G>T | c.G781T | G261C |
| c.782G>C | c.G782C | G261A |
| c.787A>C | c.A787C | N263H |
| c.788A>C | c.A788C | N263T |
| c.788A>G | c.A788G | N263S |
| c.790G>A | c.G790A | D264N |
| c.790G>C | c.G790C | D264H |
| c.790G>T | c.G790T | D264Y |
| c.793C>G | c.C793G | P265A |
| c.794C>A | c.C794A | P265Q |
| c.794C>T | c.C794T | P265L |
| c.799A>G | c.A799G | M267V |
| c.799A>T | c.A799T | M267L |
| c.800T>C | c.T800C | M267T |
| c.802T>A | c.T802A | L268I |
| c.804A>T | c.A804T | L268F |
| c.805G>A | c.G805A | V269M |
| c.805G>C | c.G805C | V269L |
| c.806T>C | c.T806C | V269A |
| c.808A>C | c.A808C | I270L |
| c.808A>G | c.A808G | I270V |
| c.809T>C | c.T809C | I270T |
| c.809T>G | c.T809G | I270S |
| c.810T>G | c.T810G | I270M |
| c.811G>A | c.G811A | G271S |
| c.[811G>A; 937G>T] | c.G811A/G937T | G271S/D313Y |
| c.812G>A | c.G812A | G271D |
| c.812G>C | c.G812C | G271A |
| c.814A>G | c.A814G | N272D |
| c.818T>A | c.T818A | F273Y |
| c.823C>A | c.C823A | L275I |
| c.823C>G | c.C823G | L275V |
| c.827G>A | c.G827A | S276N |
| c.827G>C | c.G827C | S276T |
| c.829T>G | c.T829G | W277G |
| c.830G>T | c.G830T | W277L |
| c.831G>T or c.831G>C | c.G831T or c.G831C | W277C |
| c.832A>T | c.A832T | N278Y |
| c.833A>T | c.A833T | N278I |
| c.835C>G | c.C835G | Q279E |
| c.838C>A | c.C838A | Q280K |
| c.839A>G | c.A839G | Q280R |
| c.839A>T | c.A839T | Q280L |
| c.840A>T or c.840A>C | c.A840T or c.A840C | Q280H |
| c.841G>C | c.G841C | V281L |
| c.842T>A | c.T842A | V281E |
| c.842T>C | c.T842C | V281A |
| c.842T>G | c.T842G | V281G |
| c.844A>G | c.A844G | T282A |
| c.844A>T | c.A844T | T282S |
| c.845C>T | c.C845T | T282I |
| c.847C>G | c.C847G | Q283E |
| c.848A>T | c.A848T | Q283L |
| c.849G>C | c.G849C | Q283H |
| c.850A>G | c.A850G | M284V |
| c.850A>T | c.A850T | M284L |
| c.851T>C | c.T851C | M284T |
| c.852G>C | c.G852C | M284I |
| c.853G>A | c.G853A | A285T |
| c.854C>G | c.C854G | A285G |
| c.854C>T | c.C854T | A285V |
| c.856C>G | c.C856G | L286V |
| c.856C>T | c.C856T | L286F |
| c.857T>A | c.T857A | L286H |
| c.860G>T | c.G860T | W287L |
| c.862G>C | c.G862C | A288P |
| c.862G>T | c.G862T | A288S |
| c.863C>G | c.C863G | A288G |
| c.863C>T | c.C863T | A288V |
| c.865A>C | c.A865C | I289L |
| c.865A>G | c.A865G | I289V |
| c.866T>C | c.T866C | I289T |
| c.866T>G | c.T866G | I289S |
| c.868A>C or c.868A>T | c.A868C or c.A868T | M290L |
| c.868A>G | c.A868G | M290V |
| c.869T>C | c.T869C | M290T |
| c.870G>A or c.870G>C or c.870G>T | c.G870A or c.G870C or c.G870T | M290I |
| c.871G>A | c.G871A | A291T |
| c.871G>T | c.G871T | A291S |
| c.872C>G | c.C872G | A291G |
| c.874G>T | c.G874T | A292S |
| c.875C>G | c.C875G | A292G |
| c.877C>A | c.C877A | P293T |
| c.880T>A | c.T880A | L294I |
| c.880T>G | c.T880G | L294V |
| c.881T>C | c.T881C | L294S |
| c.882A>T | c.A882T | L294F |
| c.883T>A | c.T883A | F295I |
| c.883T>G | c.T883G | F295V |
| c.884T>A | c.T884A | F295Y |
| c.884T>C | c.T884C | F295S |
| c.884T>G | c.T884G | F295C |
| c.886A>G | c.A886G | M296V |
| c.886A>T or c.886A>C | c.A886T or c.A886C | M296L |
| c.887T>C | c.T887C | M296T |
| c.888G>A or c.888G>T or c.888G>C | c.G888A or c.G888T or c.G888C | M296I |
| c.889T>A | c.T889A | S297T |
| c.892A>G | c.A892G | N298D |
| c.893A>C | c.A893C | N298T |
| c.893A>G | c.A893G | N298S |
| c.893A>T | c.A893T | N298I |
| c.895G>A | c.G895A | D299N |
| c.895G>C | c.G895C | D299H |
| c.897C>G or c.897C>A | c.C897G or c.C897A | D299E |
| c.898C>A | c.C898A | L300I |
| c.898C>G | c.C898G | L300V |
| c.898C>T | c.C898T | L300F |
| c.899T>C | c.T899C | L300P |
| c.901C>G | c.C901G | R301G |
| c.902G>A | c.G902A | R301Q |
| c.902G>C | c.G902C | R301P |
| c.902G>T | c.G902T | R301L |
| c.904C>A | c.C904A | H302N |
| c.904C>G | c.C904G | H302D |
| c.904C>T | c.C904T | H302Y |
| c.905A>T | c.A905T | H302L |
| c.907A>G | c.A907G | I303V |
| c.907A>T | c.A907T | I303F |
| c.908T>A | c.T908A | I303N |
| c.908T>C | c.T908C | I303T |
| c.908T>G | c.T908G | I303S |
| c.911G>A | c.G911A | S304N |
| c.911G>C | c.G911C | S304T |
| c.911G>T | c.G911T | S304I |
| c.916C>G | c.C916G | Q306E |
| c.917A>C | c.A917C | Q306P |
| c.917A>T | c.A917T | Q306L |
| c.919G>A | c.G919A | A307T |
| c.919G>C | c.G919C | A307P |
| c.919G>T | c.G919T | A307S |
| c.920C>A | c.C920A | A307D |
| c.920C>G | c.C920G | A307G |
| c.920C>T | c.C920T | A307V |
| c.922A>C | c.A922C | K308Q |
| c.922A>G | c.A922G | K308E |
| c.923A>G | c.A923G | K308R |
| c.923A>T | c.A923T | K308I |
| c.924A>T or c.924A>C | c.A924T or c.A924C | K308N |
| c.925G>A | c.G925A | A309T |
| c.925G>C | c.G925C | A309P |
| c.926C>A | c.C926A | A309D |
| c.926C>T | c.C926T | A309V |
| c.928C>A | c.C928A | L310I |
| c.928C>G | c.C928G | L310V |
| c.928C>T | c.C928T | L310F |
| c.931C>A | c.C931A | L311I |
| c.931C>G | c.C931G | L311V |
| c.934C>A | c.C934A | Q312K |
| c.934C>G | c.C934G | Q312E |
| c.935A>G | c.A935G | Q312R |
| c.935A>T | c.A935T | Q312L |
| c.936G>T or c.936G>C | c.G936T or c.G936C | Q312H |
| c.937G>T | c.G937T | D313Y |
| c.[937G>T; 1232G>A] | c.G937T/G1232A | D313Y/G411D |
| c.938A>G | c.A938G | D313G |
| c.938A>T | c.A938T | D313V |
| c.939T>A | c.T939A | D313E |
| c.940A>G | c.A940G | K314E |
| c.941A>C | c.A941C | K314T |
| c.941A>T | c.A941T | K314M |
| c.942G>C | c.G942C | K314N |
| c.943G>A | c.G943A | D315N |
| c.943G>C | c.G943C | D315H |
| c.943G>T | c.G943T | D315Y |
| c.944A>C | c.A944C | D315A |
| c.944A>G | c.A944G | D315G |
| c.944A>T | c.A944T | D315V |
| c.946G>A | c.G946A | V316I |
| c.946G>C | c.G946C | V316L |
| c.947T>C | c.T947C | V316A |
| c.947T>G | c.T947G | V316G |
| c.949A>C | c.A949C | I317L |
| c.949A>G | c.A949G | I317V |
| c.950T>C | c.T950C | I317T |
| c.951T>G | c.T951G | I317M |
| c.952G>A | c.G952A | A318T |
| c.952G>C | c.G952C | A318P |
| c.953C>A | c.C953A | A318D |
| c.953C>T | c.C953T | A318V |
| c.955A>T | c.A955T | I319F |
| c.956T>C | c.T956C | I319T |
| c.957C>G | c.C957G | I319M |
| c.958A>C | c.A958C | N320H |
| c.959A>C | c.A959C | N320T |
| c.959A>G | c.A959G | N320S |
| c.959A>T | c.A959T | N320I |
| c.961C>A | c.C961A | Q321K |
| c.962A>G | c.A962G | Q321R |
| c.962A>T | c.A962T | Q321L |
| c.963G>C or c.963G>T | c.G963C or c.G963T | Q321H |
| c.964G>A | c.G964A | D322N |
| c.964G>C | c.G964C | D322H |
| c.965A>C | c.A965C | D322A |
| c.965A>T | c.A965T | D322V |
| c.966C>A or c.966C>G | c.C966A or c.C966G | D322E |
| c.967C>A | c.C967A | P323T |
| c.968C>G | c.C968G | P323R |
| c.970T>G | c.T970G | L324V |
| c.971T>G | c.T971G | L324W |
| c.973G>A | c.G973A | G325S |
| c.973G>C | c.G973C | G325R |
| c.973G>T | c.G973T | G325C |
| c.974G>C | c.G974C | G325A |
| c.974G>T | c.G974T | G325V |
| c.976A>C | c.A976C | K326Q |
| c.976A>G | c.A976G | K326E |
| c.977A>C | c.A977C | K326T |
| c.977A>G | c.A977G | K326R |
| c.977A>T | c.A977T | K326M |
| c.978G>C or c.978G>T | c.G978C or c.G978T | K326N |
| c.979C>G | c.C979G | Q327E |
| c.980A>C | c.A980C | Q327P |
| c.980A>T | c.A980T | Q327L |
| c.981A>T | c.A981T | Q327H |
| c.983G>C | c.G983C | G328A |
| c.985T>A | c.T985A | Y329N |
| c.985T>C | c.T985C | Y329H |
| c.985T>G | c.T985G | Y329D |
| c.986A>G | c.A986G | Y329C |
| c.986A>T | c.A986T | Y329F |
| c.988C>A | c.C988A | Q330K |
| c.988C>G | c.C988G | Q330E |
| c.989A>C | c.A989C | Q330P |
| c.989A>G | c.A989G | Q330R |
| c.990G>C | c.G990C | Q330H |
| c.991C>G | c.C991G | L331V |
| c.992T>A | c.T992A | L331H |
| c.992T>C | c.T992C | L331P |
| c.992T>G | c.T992G | L331R |
| c.994A>G | c.A994G | R332G |
| c.995G>C | c.G995C | R332T |
| c.995G>T | c.G995T | R332I |
| c.996A>T | c.A996T | R332S |
| c.997C>G | c.C997G | Q333E |
| c.998A>C | c.A998C | Q333P |
| c.998A>T | c.A998T | Q333L |
| c.1000G>C | c.G1000C | G334R |
| c.1001G>A | c.G1001A | G334E |
| c.1001G>T | c.G1001T | G334V |
| c.1003G>T | c.G1003T | D335Y |
| c.1004A>C | c.A1004C | D335A |
| c.1004A>G | c.A1004G | D335G |
| c.1004A>T | c.A1004T | D335V |
| c.1005C>G | c.C1005G | D335E |
| c.1006A>G | c.A1006G | N336D |
| c.1006A>T | c.A1006T | N336Y |
| c.1007A>C | c.A1007C | N336T |
| c.1007A>G | c.A1007G | N336S |
| c.1007A>T | c.A1007T | N336I |
| c.1009T>G | c.T1009G | F337V |
| c.1010T>A | c.T1010A | F337Y |
| c.1010T>C | c.T1010C | F337S |
| c.1010T>G | c.T1010G | F337C |
| c.1011T>A | c.T1011A | F337L |
| c.1012G>A | c.G1012A | E338K |
| c.1013A>C | c.A1013C | E338A |
| c.1013A>G | c.A1013G | E338G |
| c.1013A>T | c.A1013T | E338V |
| c.1014A>T | c.A1014T | E338D |
| c.1015G>A | c.G1015A | V339M |
| c.1016T>A | c.T1016A | V339E |
| c.1016T>C | c.T1016C | V339A |
| c.1021G>C | c.G1021C | E341Q |
| c.1022A>C | c.A1022C | E341A |
| c.1027C>A | c.C1027A | P343T |
| c.1027C>G | c.C1027G | P343A |
| c.1027C>T | c.C1027T | P343S |
| c.1028C>T | c.C1028T | P343L |
| c.1030C>G | c.C1030G | L344V |
| c.1030C>T | c.C1030T | L344F |
| c.1031T>G | c.T1031G | L344R |
| c.1033T>C | c.T1033C | S345P |
| c.1036G>T | c.G1036T | G346C |
| c.1037G>A | c.G1037A | G346D |
| c.1037G>C | c.G1037C | G346A |
| c.1037G>T | c.G1037T | G346V |
| c.1039T>A | c.T1039A | L347I |
| c.1043C>A | c.C1043A | A348D |
| c.1046G>C | c.G1046C | W349S |
| c.1046G>T | c.G1046T | W349L |
| c.1047G>C | c.G1047C | W349C |
| c.1048G>A | c.G1048A | A350T |
| c.1048G>T | c.G1048T | A350S |
| c.1049C>G | c.C1049G | A350G |
| c.1049C>T | c.C1049T | A350V |
| c.1052T>A | c.T1052A | V351E |
| c.1052T>C | c.T1052C | V351A |
| c.1054G>A | c.G1054A | A352T |
| c.1054G>T | c.G1054T | A352S |
| c.1055C>G | c.C1055G | A352G |
| c.1055C>T | c.C1055T | A352V |
| c.1057A>T | c.A1057T | M353L |
| c.1058T>A | c.T1058A | M353K |
| c.1058T>C | c.T1058C | M353T |
| c.1061T>A | c.T1061A | 1354K |
| c.1061T>G | c.T1061G | I354R |
| c.1063A>C | c.A1063C | N355H |
| c.1063A>G | c.A1063G | N355D |
| c.1063A>T | c.A1063T | N355Y |
| c.1064A>G | c.A1064G | N355S |
| c.1066C>G | c.C1066G | R356G |
| c.1066C>T | c.C1066T | R356W |
| c.1067G>A | c.G1067A | R356Q |
| c.1067G>C | c.G1067C | R356P |
| c.1067G>T | c.G1067T | R356L |
| c.1069C>G | c.C1069G | Q357E |
| c.1072G>C | c.G1072C | E358Q |
| c.1073A>C | c.A1073C | E358A |
| c.1073A>G | c.A1073G | E358G |
| c.1074G>T or c.1074G>C | c.G1074T or c.G1074C | E358D |
| c.1075A>C | c.A1075C | I359L |
| c.1075A>G | c.A1075G | I359V |
| c.1075A>T | c.A1075T | I359F |
| c.1076T>A | c.T1076A | I359N |
| c.1076T>C | c.T1076C | I359T |
| c.1076T>G | c.T1076G | I359S |
| c.1078G>A | c.G1078A | G360S |
| c.1078G>C | c.G1078C | G360R |
| c.1078G>T | c.G1078T | G360C |
| c.1079G>A | c.G1079A | G360D |
| c.1079G>C | c.G1079C | G360A |
| c.1082G>A | c.G1082A | G361E |
| c.1082G>C | c.G1082C | G361A |
| c.1084C>A | c.C1084A | P362T |
| c.1084C>G | c.C1084G | P362A |
| c.1084C>T | c.C1084T | P362S |
| c.1085C>A | c.C1085A | P362H |
| c.1085C>G | c.C1085G | P362R |
| c.1085C>T | c.C1085T | P362L |
| c.1087C>A | c.C1087A | R363S |
| c.1087C>G | c.C1087G | R363G |
| c.1087C>T | c.C1087T | R363C |
| c.1088G>A | c.G1088A | R363H |
| c.1088G>T | c.G1088T | R363L |
| c.1090T>C | c.T1090C | S364P |
| c.1091C>G | c.C1091G | S364C |
| c.1093T>A | c.T1093A | Y365N |
| c.1093T>G | c.T1093G | Y365D |
| c.1094A>C | c.A1094C | Y365S |
| c.1094A>T | c.A1094T | Y365F |
| c.1096A>C | c.A1096C | T366P |
| c.1096A>T | c.A1096T | T366S |
| c.1097C>A | c.C1097A | T366N |
| c.1097C>T | c.C1097T | T366I |
| c.1099A>C | c.A1099C | I367L |
| c.1099A>T | c.A1099T | I367F |
| c.1101C>G | c.C1101G | I367M |
| c.1102G>A | c.G1102A | A368T |
| c.1102G>C | c.G1102C | A368P |
| c.1103C>G | c.C1103G | A368G |
| c.1105G>A | c.G1105A | V369I |
| c.1105G>C | c.G1105C | V369L |
| c.1105G>T | c.G1105T | V369F |
| c.1106T>C | c.T1106C | V369A |
| c.1106T>G | c.T1106G | V369G |
| c.1108G>A | c.G1108A | A370T |
| c.1108G>C | c.G1108C | A370P |
| c.1109C>A | c.C1109A | A370D |
| c.1109C>G | c.C1109G | A370G |
| c.1109C>T | c.C1109T | A370V |
| c.1111T>A | c.T1111A | S371T |
| c.1112C>G | c.C1112G | S371C |
| c.1117G>A | c.G1117A | G373S |
| c.1117G>T | c.G1117T | G373C |
| c.1118G>C | c.G1118C | G373A |
| c.1120A>G | c.A1120G | K374E |
| c.1121A>C | c.A1121C | K374T |
| c.1121A>G | c.A1121G | K374R |
| c.1121A>T | c.A1121T | K374I |
| c.1123G>C | c.G1123C | G375R |
| c.1124G>A | c.G1124A | G375E |
| c.1124G>C | c.G1124C | G375A |
| c.1126G>A | c.G1126A | V376M |
| c.1126G>C | c.G1126C | V376L |
| c.1127T>A | c.T1127A | V376E |
| c.1127T>G | c.T1127G | V376G |
| c.1129G>A | c.G1129A | A377T |
| c.1129G>C | c.G1129C | A377P |
| c.1129G>T | c.G1129T | A377S |
| c.1130C>G | c.C1130G | A377G |
| c.1135A>G | c.A1135G | N379D |
| c.1136A>C | c.A1136C | N379T |
| c.1136A>T | c.A1136T | N379I |
| c.1137T>A | c.T1137A | N379K |
| c.1138C>A | c.C1138A | P380T |
| c.1138C>G | c.C1138G | P380A |
| c.1139C>A | c.C1139A | P380H |
| c.1139C>G | c.C1139G | P380R |
| c.1139C>T | c.C1139T | P380L |
| c.1142C>A | c.C1142A | A381D |
| c.1147T>A | c.T1147A | F383I |
| c.1148T>A | c.T1148A | F383Y |
| c.1148T>G | c.T1148G | F383C |
| c.1150A>T | c.A1150T | I384F |
| c.1151T>C | c.T1151C | I384T |
| c.1152C>G | c.C1152G | I384M |
| c.1153A>G | c.A1153G | T385A |
| c.1154C>T | c.C1154T | T385I |
| c.1156C>A | c.C1156A | Q386K |
| c.1157A>T | c.A1157T | Q386L |
| c.1158G>C | c.G1158C | Q386H |
| c.1159C>A | c.C1159A | L387I |
| c.1159C>T | c.C1159T | L387F |
| c.1160T>A | c.T1160A | L387H |
| c.1160T>G | c.T1160G | L387R |
| c.1162C>A | c.C1162A | L388I |
| c.1162C>G | c.C1162G | L388V |
| c.1162C>T | c.C1162T | L388F |
| c.1163T>A | c.T1163A | L388H |
| c.1163T>G | c.T1163G | L388R |
| c.1168G>A | c.G1168A | V390M |
| c.1171A>C | c.A1171C | K391Q |
| c.1171A>G | c.A1171G | K391E |
| c.1172A>C | c.A1172C | K391T |
| c.1172A>G | c.A1172G | K391R |
| c.1172A>T | c.A1172T | K391I |
| c.1173A>T | c.A1173T | K391N |
| c.1174A>G | c.A1174G | R392G |
| c.1174A>T | c.A1174T | R392W |
| c.1175G>A | c.G1175A | R392K |
| c.1175G>C | c.G1175C | R392T |
| c.1175G>T | c.G1175T | R392M |
| c.1177A>C | c.A1177C | K393Q |
| c.1177A>G | c.A1177G | K393E |
| c.1178A>C | c.A1178C | K393T |
| c.1179G>C | c.G1179C | K393N |
| c.1180C>A | c.C1180A | L394I |
| c.1181T>A | c.T1181A | L394Q |
| c.1181T>C | c.T1181C | L394P |
| c.1181T>G | c.T1181G | L394R |
| c.1183G>C | c.G1183C | G395R |
| c.1184G>A | c.G1184A | G395E |
| c.1184G>C | c.G1184C | G395A |
| c.1186T>A | c.T1186A | F396I |
| c.1186T>G | c.T1186G | F396V |
| c.1187T>G | c.T1187G | F396C |
| c.1188C>G | c.C1188G | F396L |
| c.1189T>A | c.T1189A | Y397N |
| c.1189T>C | c.T1189C | Y397H |
| c.1190A>C | c.A1190C | Y397S |
| c.1190A>G | c.A1190G | Y397C |
| c.1190A>T | c.A1190T | Y397F |
| c.1192G>A | c.G1192A | E398K |
| c.1192G>C | c.G1192C | E398Q |
| c.1193A>G | c.A1193G | E398G |
| c.1195T>A | c.T1195A | W399R |
| c.1195T>G | c.T1195G | W399G |
| c.1198A>C | c.A1198C | T400P |
| c.1198A>G | c.A1198G | T400A |
| c.1198A>T | c.A1198T | T400S |
| c.1199C>A | c.C1199A | T400N |
| c.1199C>T | c.C1199T | T400I |
| c.1201T>A | c.T1201A | S401T |
| c.1201T>G | c.T1201G | S401A |
| c.1202 1203insGACTTC | c.1202 1203insGACTTC | p.T400 S401dup |
| c.1202C>T | c.C1202T | S401L |
| c.1204A>G | c.A1204G | R402G |
| c.1204A>T | c.A1204T | R402W |
| c.1205G>C | c.G1205C | R402T |
| c.1205G>T | c.G1205T | R402M |
| c.1206G>C | c.G1206C | R402S |
| c.1207T>G | c.T1207G | L403V |
| c.1208T>C | c.T1208C | L403S |
| c.1209A>T | c.A1209T | L403F |
| c.1210A>G | c.A1210G | R404G |
| c.1211G>A | c.G1211A | R404K |
| c.1211G>C | c.G1211C | R404T |
| c.1211G>T | c.G1211T | R404I |
| c.1212A>T | c.A1212T | R404S |
| c.1213A>G | c.A1213G | S405G |
| c.1216C>G | c.C1216G | H406D |
| c.1217A>T | c.A1217T | H406L |
| c.1218C>G | c.C1218G | H406Q |
| c.1219A>T | c.A1219T | I407L |
| c.1220T>C | c.T1220C | I407T |
| c.1221A>G | c.A1221G | I407M |
| c.1222A>C | c.A1222C | N408H |
| c.1222A>G | c.A1222G | N408D |
| c.1222A>T | c.A1222T | N408Y |
| c.1223A>C | c.A1223C | N408T |
| c.1225C>A | c.C1225A | P409T |
| c.1225C>G | c.C1225G | P409A |
| c.1225C>T | c.C1225T | P409S |
| c.1226C>T | c.C1226T | P409L |
| c.1228A>G | c.A1228G | T410A |
| c.1228A>T | c.A1228T | T410S |
| c.1229C>T | c.C1229T | T410I |
| c.1231G>A | c.G1231A | G411S |
| c.1231G>T | c.G1231T | G411C |
| c.1232G>A | c.G1232A | G411D |
| c.1232G>C | c.G1232C | G411A |
| c.1232G>T | c.G1232T | G411V |
| c.1234A>C | c.A1234C | T412P |
| c.1234A>G | c.A1234G | T412A |
| c.1234A>T | c.A1234T | T412S |
| c.1235C>A | c.C1235A | T412N |
| c.1235C>T | c.C1235T | T412I |
| c.1237G>A | c.G1237A | V413I |
| c.1237G>T | c.G1237T | V413F |
| c.1238T>G | c.T1238G | V413G |
| c.1240T>G | c.T1240G | L414V |
| c.1242G>C | c.G1242C | L414F |
| c.1243C>A | c.C1243A | L415I |
| c.1244T>A | c.T1244A | L415H |
| c.1246C>G | c.C1246G | Q416E |
| c.1247A>T | c.A1247T | Q416L |
| c.1248G>C | c.G1248C | Q416H |
| c.1249C>A | c.C1249A | L417I |
| c.1252G>A | c.G1252A | E418K |
| c.1252G>C | c.G1252C | E418Q |
| c.1253A>C | c.A1253C | E418A |
| c.1253A>G | c.A1253G | E418G |
| c.1254A>T | c.A1254T | E418D |
| c.1255A>G | c.A1255G | N419D |
| c.1255A>T | c.A1255T | N419Y |
| c.1256A>C | c.A1256C | N419T |
| c.1256A>G | c.A1256G | N419S |
| c.1256A>T | c.A1256T | N419I |
| c.1258A>C | c.A1258C | T420P |
| c.1258A>T | c.A1258T | T420S |
| c.1259C>A | c.C1259A | T420K |
| c.1259C>G | c.C1259G | T420R |
| c.1261A>G | c.A1261G | M421V |
| c.1261A>T | c.A1261T | M421L |
| c.1262T>A | c.T1262A | M421K |
| c.1262T>C | c.T1262C | M421T |
| c.1262T>G | c.T1262G | M421R |
| c.1263G>C | c.G1263C | M421I |
| c.1265A>C | c.A1265C | Q422P |
| c.1267A>T | c.A1267T | M423L |
| c.1268T>A | c.T1268A | M423K |
| c.1268T>C | c.T1268C | M423T |
| c.1269G>C | c.G1269C | M423I |
| c.1271C>T | c.C1271T | S424L |
| c.1275A>C | c.A1275C | L425F |
| c.1279G>A | c.G1279A | D427N |
| c.1286T>G | c.T1286G | L429R |

### Dosing, Formulation and Administration

In one or more embodiments, the Fabry patient is administered migalastat or salt thereof at a frequency of once every other day (also referred to as "QOD"). In various embodiments, the doses described herein pertain to migalastat hydrochloride or an equivalent dose of migalastat or a salt thereof other than the hydrochloride salt. In some embodiments, these doses pertain to the free base of migalastat. In alternate embodiments, these doses pertain to a salt of migalastat. In further embodiments, the salt of migalastat is migalastat hydrochloride. The administration of migalastat or a salt of migalastat is referred to herein as "migalastat therapy".

Accordingly, in one or more embodiments, the Fabry patient is administered migalastat of salt thereof in a range of from about 15 mg to about 300 mg, from about 15 mg to about 250 mg, from about 15 mg to about 200 mg, from about 15 mg to about 150 mg or from about 15 mg to about 123 mg at a frequency of once every other day, once every three days, once every four days, once every five days, once every six days or once every seven days. In one or more embodiments, the migalastat or salt thereof is administered at a frequency of once every other day (also referred to as "QOD" or "Q48H"), every four days (also referred to as "Q4D" or "Q96H") or every seven days (also referred to as "Q7D" or "Q168H"). In some embodiments, dosing intervals may include any dosing interval with more than 48 hours between doses. For example, dosing intervals may include dosing every 72, 96, 120, 144, or 168 hours.

In one or more embodiments, the Fabry patient is administered migalastat FBE in a range of from about 15 mg to about 300 mg, from about 15 mg to about 250 mg, from about 15 mg to about 200 mg, from about 15 mg to about 150 mg, from about 15 mg to about 123 mg, from about 15 mg to about 100 mg, from about 15 mg to about 50 mg, from about 50 mg to about 300 mg, from about 50 mg to about 250 mg, from about 50 mg to about 200 mg, from about 50 mg to about 150 mg, from about 50 mg to about 123 mg, from about 50 mg to about 100 mg, from about 100 mg to about 300 mg, from about 100 mg to about 250 mg, from about 100 mg to about 200 mg, from about 100 mg to about 150 mg, from about 100 mg to about 123 mg, from about 150 mg to about 300 mg, from about 150 mg to about 250 mg, from about 150 mg to about 200 mg, from about 200 mg to about 300 mg, from about 200 mg to about 250 mg or from about 250 mg to about 300 mg at a frequency of once every other day, once every three days, once every four days, once every five days, once every six days or once every seven days.

In one or more embodiments, the Fabry patient is administered migalastat FBE of about 15 mg, about 20 mg, about 25 mg, about 30 mg, about 35 mg, about 40 mg, about 45 mg, about 50 mg, about 55 mg, about 60 mg, about 65 mg, about 70 mg, about 75 mg, about 80 mg, about 85 mg, about 90 mg, about 95 mg, about 100 mg, about 105 mg, about 110 mg, about 115 mg, about 120 mg, about 123 mg, about 125 mg, about 130 mg, about 135 mg, about 140 mg, about 145 mg, about 150 mg, about 155 mg, about 160 mg, about 165 mg, about 170 mg, about 175 mg, about 180 mg, about 185 mg, about 190 mg, about 195 mg, about 200 mg, about 205 mg, about 210 mg, about 215 mg, about 220 mg, about 225 mg, about 230 mg, about 235 mg, about 240 mg, about 245 mg, about 250 mg, about 255 mg, about 260 mg, about 265 mg, about 270 mg, about 275 mg, about 280 mg, about 285 mg, about 290 mg, about 295 mg or about 300 mg at a frequency of once every other day, once every three days, once every four days, once every five days, once every six days or once every seven days.

Again, it is noted that 150 mg of migalastat hydrochloride is equivalent to 123 mg of the free base form of migalastat. Thus, in one or more embodiments, the dose is 150 mg of migalastat hydrochloride or an equivalent dose of migalastat or a salt thereof other than the hydrochloride salt, administered at a frequency of once every other day, once every three days, once every four days, once every five days, once every six days or once every seven days. In further embodiments, the dose is 150 mg of migalastat hydrochloride administered at a frequency of once every other day. In other embodiments, the dose is 123 mg of the migalastat free base administered at a frequency of once every other day.

In one or more embodiments, the Fabry patient is administered migalastat hydrochloride in a range of from about 15 mg to about 300 mg, from about 15 mg to about 250 mg, from about 15 mg to about 200 mg, from about 15 mg to about 150 mg, from about 15 mg to about 123 mg, from about 15 mg to about 100 mg, from about 15 mg to about 50 mg, from about 50 mg to about 300 mg, from about 50 mg to about 250 mg, from about 50 mg to about 200 mg, from about 50 mg to about 150 mg, from about 50 mg to about 123 mg, from about 50 mg to about 100 mg, from about 100 mg to about 300 mg, from about 100 mg to about 250 mg, from about 100 mg to about 200 mg, from about 100 mg to about 150 mg, from about 100 mg to about 123 mg, from about 150 mg to about 300 mg, from about 150 mg to about 250 mg, from about 150 mg to about 200 mg, from about 200 mg to about 300 mg, from about 200 mg to about 250 mg or from about 250 mg to about 300 mg at a frequency of once every other day, once every three days, once every four days, once every five days, once every six days or once every seven days.

In one or more embodiments, the Fabry patient is administered migalastat hydrochloride of about 15 mg, about 20 mg, about 25 mg, about 30 mg, about 35 mg, about 40 mg, about 42 mg, about 45 mg, about 50 mg, about 55 mg, about 57 mg, about 60 mg, about 65 mg, about 67 mg, about 70 mg, about 75 mg, about 77 mg, about 79 mg, about 80 mg, about 85 mg, about 90 mg, about 94 mg, about 95 mg, about 97 mg, about 100 mg, about 105 mg, about 110 mg, about 115 mg, about 120 mg, about 125 mg, about 128 mg, about 130 mg, about 135 mg, about 140 mg, about 144 mg, about 145 mg, about 150 mg, about 155 mg, about 160 mg, about 165 mg, about 170 mg, about 175 mg, about 180 mg, about 185 mg, about 190 mg, about 195 mg, about 200 mg, about 205 mg, about 210 mg, about 215 mg, about 220 mg, about 225 mg, about 230 mg, about 235 mg, about 240 mg, about 245 mg, about 250 mg, about 255 mg, about 260 mg, about 265 mg, about 270 mg, about 275 mg, about 280 mg, about 285 mg, about 290 mg, about 295 mg or about 300 mg at a frequency of once every other day, once every three days, once every four days, once every five days, once every six days or once every seven days.

In some embodiments, the patient weighs in a range of from about 10 kg to about ≥50 kg, from about 10 kg to about ≤50 kg, from about 10 kg to about ≤45 kg, from about 10 kg to about ≤40 kg, from about 10 kg to about ≤35 kg, from about 10 kg to about ≤30 kg, from about 10 kg to about ≤25 kg, from about 10 kg to about ≤20 kg, from about 10 kg to about ≤15 kg, from about 15 kg to about ≥50 kg, from about 15 kg to about ≤50 kg, from about 15 kg to about ≤45 kg, from about 15 kg to about ≤40 kg, from about 15 kg to about ≤35 kg, from about 15 kg to about ≤30 kg, from about 15 kg to about ≤25 kg, from about 20 kg to about ≥50 kg, from about 20 kg to about ≤50 kg, from about 20 kg to about ≤45 kg, from about 20 kg to about ≤40 kg, from about 20 kg to about ≤35 kg, from about 20 kg to about ≤30 kg, from about 20 kg to about ≤25 kg, from about 25 kg to about ≥50 kg, from about 25 kg to about ≤50 kg, from about 25 kg to about ≤45 kg, from about 25 kg to about ≤40 kg, from about 25 kg to about ≤35 kg, from about 25 kg to about ≤30 kg, from about 30 kg to about ≥50 kg, from about 30 kg to about ≤50 kg, from about 30 kg to about ≤45 kg, from about 30 kg to about ≤40 kg, from about 30 kg to about ≤35 kg, from about 35 kg to about ≥50 kg, from about 35 kg to about ≤50 kg, from about 35 kg to about ≤45 kg, from about 35 kg to about ≤40 kg, from about 40 kg to about ≥50 kg, from about 40 kg to about ≤50 kg, from about 40 kg to about ≤45 kg, from about 45 kg to about ≥50 kg or from about 45 kg to about ≤50 kg.

Administration of migalastat or salt thereof according to the present invention may be in a formulation suitable for any route of administration, but is preferably administered in an oral dosage form such as a tablet, capsule or solution. For example, the patient is orally administered capsules each containing 25 mg, 40 mg, 50 mg, 60 mg, 75 mg, 80 mg, 100 mg or 150 mg migalastat hydrochloride (i.e. 1-deoxygalactonojirimycin hydrochloride) or an equivalent dose of migalastat or a salt thereof other than the hydrochloride salt. In another example, the patient is orally administered capsules each containing 150 mg migalastat hydrochloride or an equivalent dose of migalastat or a salt thereof other than the hydrochloride salt.

In various embodiments, the doses described herein pertain to migalastat hydrochloride or an equivalent dose of migalastat or a salt thereof other than the hydrochloride salt. In some embodiments, these doses pertain to the free base of migalastat. In alternate embodiments, these doses pertain to a salt of migalastat. In further embodiments, the salt of migalastat is migalastat hydrochloride. The administration of migalastat or a salt of migalastat is referred to herein as "migalastat therapy".

The administration of migalastat or salt thereof may be for a certain period of time. In one or more embodiments, the migalastat or salt thereof is administered for a duration of at least 28 days, such as at least 30, 60 or 90 days or at least 4, 6, 8, 12, 16, 26 or 52 weeks or at least 1,2, 3, 4, 5, 6, 7, 8, 9, 10, 11, 12, 16, 20, 24, 30 or 36 months or at least 1, 2, 3, 4 or 5 years. In some embodiments, the migalastat therapy is of at least about 4 weeks. In various embodiments, the migalastat therapy is a long-term migalastat therapy of at least about 2, 3, 4 or 5 years.

In some embodiments, the PC (*e.g.,* migalastat or salt thereof) is administered orally. In one or more embodiments, the PC (*e.g.,* migalastat or salt thereof) is administered by injection. The PC may be accompanied by a pharmaceutically acceptable carrier, which may depend on the method of administration.

In one or more embodiments, the PC (*e.g.,* migalastat or salt thereof) is administered as monotherapy, and can be in a form suitable for any route of administration, including *e.g.,* orally in the form tablets or capsules or liquid, or in sterile aqueous solution for injection. In other embodiments, the PC is provided in a dry lyophilized powder to be added to the formulation of the replacement enzyme during or immediately after reconstitution to prevent enzyme aggregation *in vitro* prior to administration.

When the PC (*e.g.,* migalastat or salt thereof) is formulated for oral administration, the tablets or capsules can be prepared by conventional means with pharmaceutically acceptable excipients such as binding agents (*e.g.,* pregelatinized maize starch, polyvinylpyrrolidone or hydroxypropyl methylcellulose); fillers (*e.g.,* lactose, microcrystalline cellulose or calcium hydrogen phosphate); lubricants (*e.g.,* magnesium stearate, talc or silica); disintegrants (*e.g.,* potato starch or sodium starch glycolate); or wetting agents (*e.g.,* sodium lauryl sulfate). The tablets may be coated by methods well known in the art. Liquid preparations for oral administration may take the form of, for example, solutions, syrups or suspensions, or they may be presented as a dry product for constitution with water or another suitable vehicle before use. Such liquid preparations may be prepared by conventional means with pharmaceutically acceptable additives such as suspending agents (*e.g.,* sorbitol syrup, cellulose derivatives or hydrogenated edible fats); emulsifying agents (*e.g.,* lecithin or acacia); non-aqueous vehicles (*e.g.,* almond oil, oily esters, ethyl alcohol or fractionated vegetable oils); and preservatives (*e.g.,* methyl or propyl-p- hydroxybenzoates or sorbic acid). The preparations may also contain buffer salts, flavoring, coloring and sweetening agents as appropriate. Preparations for oral administration may be suitably formulated to give controlled release of the active chaperone compound.

The pharmaceutical formulations of the PC (*e.g.,* migalastat or salt thereof) suitable for parenteral/injectable use generally include sterile aqueous solutions (where water soluble), or dispersions and sterile powders for the extemporaneous preparation of sterile injectable solutions or dispersion. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and polyethylene glycol, and the like), suitable mixtures thereof, and vegetable oils. The proper fluidity can be maintained, for example, by the use of a coating such as lecithin, by the maintenance of the required particle size in the case of dispersion and by the use of surfactants. Prevention of the action of microorganisms can be brought about by various antibacterial and antifungal agents, for example, parabens, chlorobutanol, phenol, benzyl alcohol, sorbic acid, and the like. In many cases, it will be reasonable to include isotonic agents, for example, sugars or sodium chloride. Prolonged absorption of the injectable compositions can be brought about by the use in the compositions of agents delaying absorption, for example, aluminum monosterate and gelatin.

Sterile injectable solutions are prepared by incorporating the purified enzyme (if any) and the PC (*e.g.,* migalastat or salt thereof) in the required amount in the appropriate solvent with various of the other ingredients enumerated above, as required, followed by filter or terminal sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum drying and the freeze-drying technique which yield a powder of the active ingredient plus any additional desired ingredient from previously sterile-filtered solution thereof.

The formulation can contain an excipient. Pharmaceutically acceptable excipients which may be included in the formulation are buffers such as citrate buffer, phosphate buffer, acetate buffer, bicarbonate buffer, amino acids, urea, alcohols, ascorbic acid, and phospholipids; proteins, such as serum albumin, collagen, and gelatin; salts such as EDTA or EGTA, and sodium chloride; liposomes; polyvinylpyrollidone; sugars, such as dextran, mannitol, sorbitol, and glycerol; propylene glycol and polyethylene glycol (*e.g.,* PEG-4000, PEG-6000); glycerol; glycine or other amino acids; and lipids. Buffer systems for use with the formulations include citrate; acetate; bicarbonate; and phosphate buffers. Phosphate buffer is a preferred embodiment.

The route of administration of the chaperone compound may be oral or parenteral, including intravenous, subcutaneous, intra-arterial, intraperitoneal, ophthalmic, intramuscular, buccal, rectal, vaginal, intraorbital, intracerebral, intradermal, intracranial, intraspinal, intraventricular, intrathecal, intracisternal, intracapsular, intrapulmonary, intranasal, transmucosal, transdermal, or via inhalation.

Administration of the above-described parenteral formulations of the chaperone compound may be by periodic injections of a bolus of the preparation, or may be administered by intravenous or intraperitoneal administration from a reservoir which is external (*e.g.,* an i.v. bag) or internal (*e.g.,* a bioerodable implant).

Embodiments relating to pharmaceutical formulations and administration may be combined with any of the other embodiments of the invention, for example embodiments relating to methods of treating patients with Fabry disease, methods of treating ERT-naive Fabry patients, methods of treating ERT-experienced Fabry patients, methods of reducing the risk of CBV events, methods of reducing the risk of composite clinical outcomes, methods of assessing symptoms or outcomes of a patient or groups of patients, methods of evaluating a treatment therapy, methods of enhancing α-Gal A in a patient diagnosed with or suspected of having Fabry disease, use of a pharmacological chaperone for α-Gal A for the manufacture of a medicament for treating a patient diagnosed with Fabry disease or to a pharmacological chaperone for α-Gal A for use in treating a patient diagnosed with Fabry disease as well as embodiments relating to amenable mutations, the PCs and suitable dosages thereof.

In one or more embodiments, the PC (*e.g.,* migalastat or salt thereof) is administered in combination with ERT. ERT increases the amount of protein by exogenously introducing wild-type or biologically functional enzyme by way of infusion. This therapy has been developed for many genetic disorders, including LSDs such as Fabry disease, as referenced above. After the infusion, the exogenous enzyme is expected to be taken up by tissues through non-specific or receptor-specific mechanism. In general, the uptake efficiency is not high, and the circulation time of the exogenous protein is short. In addition, the exogenous protein is unstable and subject to rapid intracellular degradation as well as having the potential for adverse immunological reactions with subsequent treatments. In one or more embodiments, the chaperone is administered at the same time as replacement enzyme (*e.g.,* replacement α-Gal A). In some embodiments, the chaperone is co-formulated with the replacement enzyme (*e.g.,* replacement α-Gal A).

In one or more embodiments, a patient is switched from ERT to migalastat therapy. In some embodiments, a patient on ERT is identified, the patient's ERT is discontinued, and the patient begins receiving migalastat therapy. The migalastat therapy can be in accordance with any of the methods described herein. In various embodiments, the patient has some degree of renal impairment, such as mild, moderate or severe renal impairment.

### Administration of Migalastat

In some embodiments, migalastat or salt thereof is administered to an adult patient. In some embodiments, age of the adult patient is ≥18 years. In some embodiments, migalastat or salt thereof is administered to an adolescent patient. In some embodiments, age of the adolescent patient is in a range of from 12 years to <18 years, from 13 years to <18 years, from 14 years to <18 years, from 15 years to <18 years, from 16 years to <18 years, from 17 years to <18 years, from 12 years to ≤17 years, from 13 years to ≤17 years, from 14 years to ≤17 years, from 15 years to ≤17 years, from 16 years to ≤17 years, from 12 years to ≤16 years, from 13 years to ≤16 years, from 14 years to ≤16 years, from 15 years to ≤16 years, from 12 years to ≤15 years, from 13 years to ≤15 years, from 14 years to ≤15 years, from 12 years to <14 years, from 13 years to ≤14 years, or from 12 years to ≤13 years.

In some embodiments, migalastat or salt thereof is administered to the patient having a weight a range of from <15 kg to ≥45 kg, from 15 kg to <25 kg, from 25 kg to <35 kg, or from 35 kg to <45 kg. In some embodiments, migalastat or salt thereof is administered to the patient having a weight <15 kg. In some embodiments, migalastat or salt thereof is administered to the patient having a weight ≥45 kg.

In some embodiments, about 25 mg of migalastat or salt thereof is administered to the patient having a weight of <15 kg. In some embodiments, about 50 mg of migalastat or salt thereof is administered to the patient having a weight in a range of from 15 kg to <25 kg. In some embodiments, about 75 mg of migalastat or salt thereof is administered to the patient having a weight in a range of from 25 kg to <35 kg. In some embodiments, about 75 mg of migalastat or salt thereof is administered to the patient having a weight in a range of from 35 kg to <50 kg.

In some embodiments, the migalastat or salt thereof is administered at a first frequency for a first time period, and then administered at a second frequency for a second time period. The first frequency is greater (i.e., more frequent) than the second frequency. The first frequency and the second frequency may be any dosing interval disclosed herein. In some embodiments, the first frequency is every other day and the second frequency is every three days, every four days, every five days, every six days or every seven days. In some embodiments, the first frequency is every four days and the second frequency is every five days, every six days, or every seven days.

In some embodiments, the migalastat or salt thereof is administered at a first frequency for a first time period, then administered at a second frequency for a second time period, and then administered at a third frequency for a third time period. The first frequency is greater (i.e., more frequent) than the second frequency, and the second frequency is greater than the third frequency. For example, in some embodiments, the migalastat or salt thereof is administered at a first frequency of once every other day for a first time period, then the migalastat or salt thereof is administered at a second frequency of once every four days for a second time period, and then the migalastat or salt thereof is administered at a third frequency of once every seven days for a third time period.

### Administration of Migalastat without Caffeine

As mentioned above and described in further detail in the Examples below, caffeine was surprisingly found to have a significant effect on the pharmacokinetics of migalastat. Accordingly, in some embodiments the patient does not consume caffeine within a certain time interval of administering the formulation comprising migalastat or a salt thereof. In various embodiments, this time interval includes abstaining from caffeine for at least 30 minutes, at least 60 minutes (1 hour), at least 90 minutes (1.5 hours), at least 2 hours, at least 2.5 hours, at least 3 hours or at least 4 hours prior to administering the migalastat or salt thereof and at least 30 minutes, at least 60 minutes (1 hour), at least 90 minutes (1.5 hours), at least 2 hours, at least 2.5 hours, at least 3 hours or at least 4 hours after administering the migalastat or salt thereof.

In some embodiments, the patient does not consume caffeine within a time interval from at least 1 hour prior to and at least 1 hour after administering the migalastat or salt thereof, i.e. the patient does not consume caffeine within about 1 hour of administering the formulation comprising migalastat or a salt thereof.

In some embodiments, the patient does not consume caffeine within a time interval from at least 2 hours prior to and at least 1 hour after administering the migalastat or salt thereof.

In some embodiments, the patient does not consume caffeine within a time interval from at least 2 hours prior to and at least 2 hours after administering the migalastat or salt thereof, i.e. the patient does not consume caffeine within about 2 hours of administering the formulation comprising migalastat or a salt thereof.

In some embodiments, the patient does not consume caffeine within a time interval from at least 3 hours prior to and at least 2 hours after administering the migalastat or salt thereof.

In some embodiments, the patient does not consume caffeine within a time interval from at least 3 hours prior to and at least 3 hours after administering the migalastat or salt thereof, i.e. the patient does not consume caffeine within about 3 hours of administering the formulation comprising migalastat or a salt thereof.

In some embodiments, the patient consumes caffeine outside of the time interval for abstaining from caffeine. For example, if the time interval for abstaining from caffeine is at least 2 hours prior to and at least 2 hours after administering the migalastat or salt thereof, then in some embodiments the patient consumes caffeine at least 2 hours prior to and/or at least 2 hours after administering the migalastat or salt thereof. In various embodiments, the patient consumes caffeine at least 30 minutes, at least 60 minutes (1 hour), at least 90 minutes (1.5 hours), at least 2 hours, at least 2.5 hours, at least 3 hours or at least 4 hours prior to administering the migalastat or salt thereof. In various embodiments, the patient consumes caffeine at least 30 minutes, at least 60 minutes (1 hour), at least 90 minutes (1.5 hours), at least 2 hours, at least 2.5 hours, at least 3 hours or at least 4 hours after administering the migalastat or salt thereof.

In some embodiments, not consuming caffeine within a certain time interval of administering the formulation comprising migalastat or a salt thereof provides improvements in the pharmacokinetics of migalastat, such as avoiding a decrease in migalastat area under the curve (AUC) and/or maximum plasma concentration (Cₘₐₓ). In some embodiments, the patient does not consume caffeine within 2 hours of administering the formulation comprising migalastat or a salt thereof to avoid a decrease in AUC and Cₘₐₓ for migalastat of about 57% and about 60%, respectively.

In some embodiments, the patient fasts during the time interval for abstaining from caffeine. In some embodiments, the patient does not consume food for at least 2 hours before and at least 2 hours after administering the migalastat or salt thereof and the patient does not consume caffeine for at least 2 hours before and at least 2 hours after administering the migalastat or salt thereof.

In some embodiments, the patient fasts for a different time interval than the time interval for abstaining from caffeine.

In some embodiments, the patient does not consume caffeinated beverages during the time interval. In some embodiments, the caffeinated beverages include coffee, espresso, tea, caffeinated energy drinks and caffeinated sodas.

In some embodiments, the patient consumes non-caffeinated beverages during the time interval that caffeine is not consumed. Examples of suitable non-caffeinated beverages include, but are not limited to, water (plain, flavored, sweetened), fruit juices without pulp, and caffeine-free carbonated beverages. In some embodiments, the non-caffeinated beverage includes a sweetened beverage. In some embodiments, the non-caffeinated beverage comprises an artificially sweetened beverage. In some embodiments, the artificial sweetener comprises aspartame or acesulfame potassium. Other artificial sweeteners and/or sugar substitutes include, but are not limited to, sucralose, stevia and saccharin. In some embodiments, non-caffeinated and/or low caffeinated beverages include decaffeinated coffee or decaffeinated tea.

In some embodiments, rather than a complete abstention from caffeine, the patient consumes only a small amount of caffeine during the time interval that caffeine is not consumed. In various embodiments, the patient limits total caffeine intake to less than 200 mg, less than 190 mg, less than 180 mg, less than 170 mg, less than 160 mg, less than 150 mg, less than 140 mg, less than 130 mg, less than 120 mg, less than 110 mg, less than 100 mg, less than 95 mg, less than 90 mg, less than 85 mg, less than 80 mg, less than 75 mg, less than 70 mg, less than 65 mg, less than 60 mg, less than 55 mg, less than 50 mg, less than 45 mg, less than 40 mg, less than 35 mg, less than 30 mg, less than 25 mg, less than 20 mg, less than 15 mg, less than 10 mg, less than 5 mg, less than 4 mg, less than 3 mg, less than 2 mg or less than 1 mg during the time interval that caffeine is not consumed.

Other aspects of the present invention relate to informing patients about the effect of caffeine consumption on migalastat pharmacokinetics (e.g. AUC and Cₘₐₓ) and/or instructing patients that caffeine should not be consumer within a certain time interval of the migalastat administration. In some embodiments, this information and/or instruction is orally provided to the patient by a health care provider. In some embodiments, this information and/or instructions is provided to the patient in written form, such as in the prescribing information, product label, product characteristics, product monograph, patient information or the like. In various embodiments, this information and/or instructions is provided in the product characteristics and/or prescribing information for GALAFOLD^{®} in various countries in which GALAFOLD^{®} is approved for use, or at a website such as www.galafoldamenabilitytable.com or www.fabrygenevariantsearch.com, each of which is hereby incorporated by reference in its entirety.

In some embodiments, the information and/or instructions include one or more of the following:
- A pharmacokinetic study showed that administration of coffee containing approximately 190 mg of caffeine resulted in a significant decrease in migalastat systemic exposure (mean reduction in AUC_{0-∞} by 57% and mean reduction in Cmax by 60%) when compared to water.
- A single-dose, 6-way crossover pharmacokinetic study was conducted in 20 healthy subjects to evaluate plasma the bioavailability of a 150 mg migalastat HCl capsule when administered with coffee and sweetened beverages relative to administration with water. The rate of absorption (tₘₐₓ) of migalastat was not affected by administration of coffee or sweetened beverages in comparison to water. However, consumption of 280 mL of coffee containing approximately 190 mg caffeine at the time of dosing resulted in significant decrease in migalastat systemic exposure (mean reduction in AUC_{0-∞} by 57% and mean reduction in Cₘₐₓ by 60%) when compared to water. The bioavailability of migalastat did not appreciably differ when administered with natural (sucrose: 8027 ng·h/mL AUC_{0-∞} and 1265 ng/mL Cₘₐₓ) and artificial (aspartame or acesulfame K: 9075 ng.h/mL, 8641 ng.h/mL AUC_{0-∞} and 1374 ng/mL, 1225 ng/mL Cₘₐₓ respectively) sweeteners when compared to water (8613 ng.h/mL AUC_{0-∞} and 1328 ng/mL Cₘₐₓ).
- In addition to not consuming food at least 2 hours before and 2 hours after taking migalastat, caffeine should not be consumed during this period.
- Caffeine in any form should not be consumed during the 4-hour fasting period.
- Consuming caffeine-containing beverages or other products containing caffeine could affect the way migalastat works.
- Water (plain, flavored, sweetened), fruit juices without pulp, and caffeine-free carbonated beverages can be consumed during the 4-hour fasting period.
- Migalastat exposure is decreased by approximately 40% when taken with food and therefore it should be taken on an empty stomach. Food should not be consumed at least 2 hours before and 2 hours after taking migalastat to give a minimum 4 hours fast. Clear liquids can be consumed during this period, for example water, fruit juices without pulp, carbonated drinks, tea or coffee without milk or cream.

### Monitoring Lyso-Gb3 and Migalastat Levels

Lyso-Gb3 (globotriaosylsphingosine) can be monitored to determine whether substrate is being cleared from the body of a Fabry patient. Higher levels of lyso-Gb3 correlate with higher levels of substrate. If a patient is being successfully treated, then lyso-Gb3 levels are expected to drop. One dosing regimen for Fabry disease is administering to the patient about 20 mg to about 300 mg FBE of migalastat or salt thereof at a frequency of once every other day.

In some embodiments, the method further comprises measuring migalastat levels. In one or more embodiments, migalastat concentration (*e.g.,* ng/mL) is measured. In some embodiments, the total area under the curve (AUC_{0-∞}) is measured. In one or more embodiments, the lowest concentration the migalastat reaches before the next dose (C_{trough}) is measured.

Migalastat levels can be measured via methods known in the art. For example, if measuring migalastat from tissue samples, tissue aliquots may be homogenized (7 µL water per 1 mg tissue) using a homogenizer (*e.g.,* FastPrep-24 from MP Biomedical, Irvine, CA). Microcentrifuge tubes containing 100 µl of the tissue homogenate or 50 µl of plasma may then be spiked with 500 ng/mL 13C d2-AT1001 HCl internal standard (manufactured by MDS Pharma Services). A 600 µl volume of 5 mM HCl in 95/5 MeOH:H₂O can then be added and the tubes vortexed for 2 minutes, followed by centrifugation at 21000 x g for 10 minutes at room temperature. The supernatants may then be collected into a clean, 96-well plate, diluted with 5 mM HCl in dH₂O and applied to a 96-well solid phase extraction (SPE) plate (Waters Corp., Milford MA). After several wash steps and elution into a clean, 96-well plate, the extracts may be dried down under N₂ and reconstituted with mobile phase A. Migalastat levels can then be determined by liquid chromatography - tandem mass spectroscopy (LC-MS/MS) (*e.g.,* LC: Shimadzu; MS/MS: ABSciex API 5500 MS/MS). The liquid chromatography can be conducted using an ACN:water:formate binary mobile phase system (mobile phase A: 5 mM ammonium formate, 0.5% formic acid in 95:5 ACN:water; mobile phase B: 5 mM ammonium formate, 0.5% formic acid in 5:47.5:47.5 ACN:MeOH:water) with a flow rate of 0.7 mL/minute on an Halo HILIC column (150x4.6 mm, 2.7 µm) (Advanced Materials Technology, Inc.). MS/MS analysis may be carried out under APCi positive ion mode. The same procedure may be followed for migalastat determination in plasma except without homogenization. The following precursor ion→product ion transitions may be monitored: mass/charge (m/z) 164.1→m/z 80.1 for migalastat and m/z 167.1→m/z 83.1 for the internal standard. A 12-point calibration curve and quality control samples may be prepared. The ratio of the area under the curve for migalastat to that of the internal standard is then determined and final concentrations of migalastat in each sample calculated using the linear least squares fit equation applied to the calibration curve. To derive approximate molar concentrations, one gram of tissue may be estimated as one mL of volume.

In some embodiments, samples may be taken at 0, 1, 2, 3, 4, 6, 8, 12, 24, 48, 72, 96, 120, 144 and/or 168 hours after administration. In some embodiments, the migalastat concentration 48 hours after administration is measured. In some embodiments, the administration of the second time period is begun after more than about 5, 10, 15, 20, 25, 40, 50, 60, 70, 80, 90, 100, 125, 150, 175 or 200 ng/mL of migalastat is measured 48 hours after administration of the migalastat during the first time period is measured.

In some embodiments, Lyso-Gb3 can be measured via methods known in the art using validated assays. As with migalastat, lyso-Gb3 levels may be determined using liquid chromatography - tandem mass spectroscopy (LC-MS/MS) (*e.g.,* LC: Shimadzu; MS/MS: ABSciex API 5500 MS/MS). For example, one process of measuring plasma lyso-Gb3 is described in Hamler, Rick, et al. "Accurate quantitation of plasma globotriaosylsphingosine (lyso-Gb3) in normal individuals and Fabry disease patients by liquid chromatography-tandem mass spectrometry (LC-MS/MS)." Molecular Genetics and Metabolism, Volume 114.2 (2015):S51. In one or more embodiments, lyso-Gb3 is measured in samples from a patient's urine.

### Dose Adjustment

In some embodiments, the dosing frequency of migalastat or salt thereof is adjusted in response to a change in the patient's eGFR. In exemplary embodiments, when the patient's eGFR is reduced below 60 mL/min/1.73 m², below 45 mL/min/1.73 m², below 30 mL/min/1.73 m² or below 15 mL/min/1.73 m², the dosing frequency can be reduced. In some embodiments, the patient is not administered migalastat or salt thereof, when the patient's eGFR is reduced below 60 mL/min/1.73 m², below 45 mL/min/1.73 m², below 30 mL/min/1.73 m² or below 15 mL/min/1.73 m².

Migalastat concentration can be measured from plasma samples at various times to monitor clearance from the body. A clinically relevant increase in C_{trough} suggests significant accumulation of plasma migalastat concentration. If the migalastat is not cleared from the body enough prior to the next dose administration, then the levels of migalastat can build up, possibly leading to an inhibitory effect. Thus, in one or more embodiments, a change in the dosing frequency occurs after a 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9 or 3.0-fold increase in C_{trough} compared to normal renal function C_{trough}.

In one or more embodiments, a change in the dosing frequency occurs after a 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9 or 3.0-fold increase in AUC_{0-∞} compared to normal renal function AUC_{0-∞}.

In some embodiments, the method further comprises measuring lyso-Gb3 in one or more plasma samples from the patient. A first baseline lyso-Gb3 level may be determined during the first time period. As used herein, "baseline lyso-Gb3 level" refers to the lowest plasma lyso-Gb3 value measured during a given time period or dosing regimen. Thus, if the lyso-Gb3 levels go up significantly from the baseline lyso-Gb3 levels, this may indicate kidney disease progression and/or improper clearance of migalastat. Thus, in further embodiments, the administration of the second time period is begun after an increase (e.g., of at least about 20, 25, 30, 33, 35, 40, 45 or 50% and/or 1, 1.25, 1.5, 1.75, 2, 2.25, 2.5 or 3 nM) above the first baseline lyso-Gb3 level is measured. A 33% and/or 2 nM increase from baseline in plasma lyso-Gb3 has been deemed clinically relevant based upon Phase 3 data in Fabry patients signaling either inhibition-induced migalastat exposure from decline in renal function and/or progression of disease condition. Lyso-Gb3 levels may be measured at varying frequencies (e.g., about once every 2, 3, 4 or 5 months). It is thought that it takes about 3 months for a baseline lyso-Gb3 level to be established once a dosing regimen has been started.

In some embodiments, the administration of the second time period may begin after an increase above the first baseline lyso-Gb3 level is at least about 30, or 33% and/or 2nM and/or more than about 50 ng/mL of migalastat is measured 48 hours after administration of the migalastat during the first time period is measured. In some embodiments, the administration of the second time period may begin after an increase above the first baseline lyso-Gb3 level is at least about 30, or 33% and/or 2nM and/or more than about 50 ng/mL of migalastat is measured 48 hours after administration of the migalastat during the first time period is measured, or there is a greater than 1.5-fold increase in AUC_{0-∞} and/or C_{trough} compared to normal renal function during the first time period.

### EXAMPLES

### Example 1: Study of Effect of Caffeine and Sweeteners on Migalastat Pharmacokinetics

The example describes the AT1001-045 study, which was an open-label study of the bioavailability, safety and tolerability of migalastat in combination with caffeine and sweeteners.

### Objectives and Endpoints

The primary objective was to evaluate plasma migalastat bioavailability of the 150 mg migalastat HCl capsule in a caffeinated beverage, a sucrose beverage, a combination caffeinated and sucrose beverage, an aspartame artificial sweetener beverage, and an acesulfame potassium artificial sweetener beverage relative to water in healthy subjects.

The secondary objective was to assess the safety and tolerability of migalastat HCl in healthy subjects.

The primary endpoints were ANOVA comparisons of interest between each test treatment and reference treatment for Cₘₐₓ, AUC₀₋ₜ and AUC_{0-∞}. The comparisons of interest were point estimate ratios and lower/upper 90% confidence intervals.

### Study Design

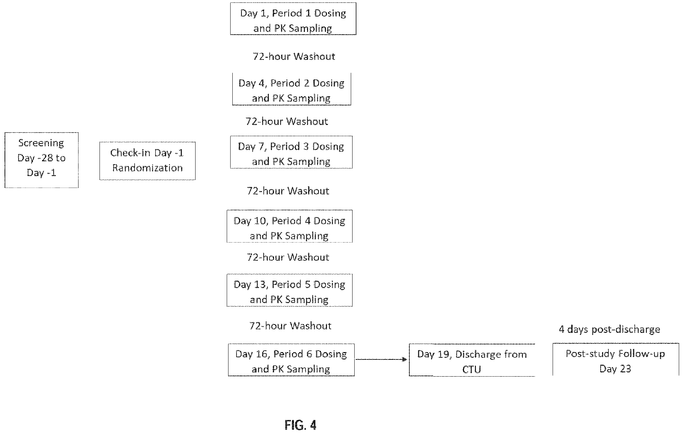

This was a single-center, single dose, randomized, open-label, 6-way crossover study. Each subject received a single oral dose of migalastat HCl 150 mg for each of 6 periods. The study schematic is shown in FIG. 4.

In randomized sequence, each subject received a 150 mg migalastat HCl capsule with a caffeinated beverage, a sucrose drink, a combination caffeinated and sucrose beverage, an aspartame artificial sweetener drink, an acesulfame potassium artificial sweetener drink, or water.

All study treatments were administered in the fasted condition (overnight + 4 hours post-dose).

Each single dose administration was followed by a 72-hour PK sampling period which also served as the between-treatment washout interval.

Subjects were domiciled for duration of the 6 treatment periods through the Period 6 72-hour blood sample (approximately 19 days, including Day -1).

Approximately 7 days after Period 6 dosing (Day 23), subjects returned to the clinic for a follow-up visit.

The entire duration of the study, including screening was approximately 7.5 weeks.

Subjects who discontinued from the study were not be replaced.

Appropriate migalastat exposure ratios (Cmax and AUC) with corresponding 90% confidence intervals will be used to address the comparisons of interest:
- Caffeine (test) vs. water (reference)
- Sucrose (test) vs. water (reference)
- Caffeine + Sucrose (test) vs. water (reference)
- Aspartame (test) vs. water (reference)
- Acesulfame K (test) vs. water (reference)

### Study Population, Sample Size and Dose

Study Population: male and female healthy subjects 18 to 45 years of age.

Sample Size: 20 subjects balanced for gender.

Dose: A single 150 mg capsule of migalastat hydrochloride, provided as GALAFOLD^{®}.

### Preparation of Test Treatments

The following test treatments were prepared:
Caffeinated beverage: 8 oz of caffeinated tea; nothing added; administered warm (40 to 50 degrees C) and consumed within 10 minutes
Sucrose drink: 8 oz sucrose solution prepared with 26 grams sucrose, chilled before drug administration, and consumed within 10 minutes
   - Equivalent to sucrose content in one 8 oz serving of cane sugar-containing Coca-Cola^{®}
Caffeinated + Sucrose drink: 8 oz caffeinated/sucrose beverage, chilled before drug administration, and consumed within 10 minutes (e.g., Jolt ^{®})
Aspartame: 8 oz aspartame solution prepared with 125 mg aspartame, chilled before drug administration, and consumed within 10 minutes
   - Equivalent to aspartame content in one 8 oz can of Diet Coke^{®}
Acesulfame K: 8 oz acesulfame K solution prepared with 30 mg acesulfame K, chilled before drug administration, and consumed within 10 minutes
   - Equivalent to acesulfame K content in one 8 oz can of Diet Coke^{®} or Coca-Cola^{®} Zero Sugar

### Results

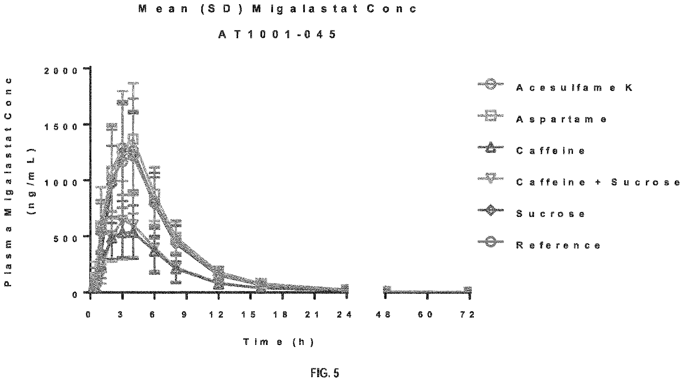

The pharmacokinetics of migalastat for each treatment are shown in FIG. 5 and Table 2 below. For a treatment to be considered bioequivalent to water, the 90% confidence intervals must be within 80% to 125% of the values with water.

**Table 2 - Migalastat Pharmacokinetics**

| Treatment | Cₘₐₓ (ng/mL) | | AUC₀₋ₜ (ng·h/mL) | | AUC_{0-∞} (ng·h/mL) | | tₘₐₓ (h) | t_{½} (h) |
|---|---|---|---|---|---|---|---|---|
| | GeoMean (CV%) [N] | Ratio (90% CI's | GeoMean (CV%) [N] | Ratio (90% CI's) | GeoMean (CV%) [N] | Ratio (90% CI's) | Median (Min - Max) [N] | Mean (CV%) [N] |
| Reference (water) | 1328 (33.4) [20] | - | 8616 (29.0) [20] | - | 8570 (30.8) [17] | - | 4 (2.0 - 4.0) [20] | 7.5 (59.2) [17] |
| Acesulfame K | 1225 (35.7) [20] | 92.3 (81.0, 105.0) | 8117 (36.5) [20] | 93.9 (82.7, 106.6) | 8621 (35.9) [15] | 96.1 (82.7, 111.8) | 4 (2.0 - 6.0) [20] | 7.2 (51.3) [15] |
| Aspartame | 1374 (33.7) [20] | 103.3 (90.7, 117.6) | 9081 (30.4) [20] | 104.9 (82.7, 106.6) | 9043 (30.5) [18] | 102.9 (89.3, 118.7) | 4 (2.0 - 4.0) [20] | 8.1 (63.3) [18] |
| Sucrose | 1265 (31.5) [20] | 95.3 (83.7, 108.5) | 7959 (29.8) [20] | 92.2 (81.2, 104.7) | 8008 (30.2) [19] | 91.8 (79.7, 105.8) | 3 (2.0 - 4.0) [20] | 7.8 (80.5) [19] |
| Caffeine | 529 (41.3) [20] | 39.9 (35.0, 45.4) | 3717 (48.3) [20] | 43.1 (38.0, 49.0) | 4100 (53.5) [15] | 45.8 (39.4, 53.4) | 3 (2.0 - 4.0) [20] | 10.2 (84.9) [15] |
| Caffeine + Sucrose | 611 (39.8) [20] | 45.9 (40.3, 52.3) | 4052 (39.3) [20] | 46.9 (41.3, 53.2) | 4268 (44.5) [15] | 47.7 (40.9, 55.5) | 3 (2.0 - 4.0) [20] | 8.8 (87.8) [15] |

As can be seen from FIG. 5 and Table 2, the artificial sweeteners acesulfame K and aspartame are bioequivalent to water. Accordingly, in some embodiments, drinks containing these artificial sweeteners may be administered with migalastat.

Sucrose was very close to bioequivalent; the mean difference in AUC was only an 8% decrease with sucrose which is not considered clinically relevant. Accordingly, in some embodiments, drinks containing sucrose may be administered with migalastat.

There was a major caffeine-migalastat interaction; Cₘₐₓ was decreased by 60%, and AUC was decreased by 57% for caffeine alone; similar decreases were observed for caffeine + sucrose. Accordingly, in some embodiments, migalastat should not be administered with caffeinated beverages.

The patent and scientific literature referred to herein establishes the knowledge that is available to those with skill in the art. All United States patents and published or unpublished United States patent applications cited herein are incorporated by reference. All published foreign patents and patent applications cited herein are hereby incorporated by reference. All other published references, documents, manuscripts and scientific literature cited herein are hereby incorporated by reference.

While this invention has been particularly shown and described with references to preferred embodiments thereof, it will be understood by those skilled in the art that various changes in form and details may be made therein without departing from the scope of the invention encompassed by the appended claims.

The embodiments described herein are intended to be illustrative of the present compositions and methods and are not intended to limit the scope of the present invention. Various modifications and changes consistent with the description as a whole and which are readily apparent to the person of skill in the art are intended to be included. The appended claims should not be limited by the specific embodiments set forth in the examples, but should be given the broadest interpretation consistent with the description as a whole.

Patents, patent applications, publications, product descriptions, GenBank Accession Numbers, and protocols are cited throughout this application, the disclosures of which are incorporated herein by reference in their entireties for all purposes.

The following items 1 to 38 represent further embodiments of the present invention:
1. A method of administering migalastat to a patient, the method comprising orally administering to the patient a formulation comprising a therapeutically effective dose of migalastat or a salt thereof, wherein the patient does not consume caffeine within about 2 hours of administering the formulation comprising migalastat or a salt thereof.
2. The method of item 1, further comprising administering caffeine to the patient at least about 2 hours prior to administering the formulation comprising migalastat or a salt thereof.
3. The method of item 1 or 2, further comprising administering caffeine to the patient at least about 2 hours after administering the formulation comprising migalastat or a salt thereof.
4. The method of any one of items 1-3, wherein the patient does not consume caffeine within 2 hours of administering the formulation comprising migalastat or a salt thereof to avoid a decrease in AUC and Cₘₐₓ for migalastat of about 57% and about 60%, respectively.
5. The method of any one of items 1-4, wherein the patient fasts for at least about 2 hours prior to administering the formulation comprising migalastat or a salt thereof and for at least about 2 hours after administering the formulation comprising migalastat or a salt thereof.
6. The method of any one of items 1-5, wherein the patient does not consume caffeine for at least about 3 hours prior to administering the formulation comprising migalastat or a salt thereof and for at least about 2 hours after administering the formulation comprising migalastat or a salt thereof.
7. The method of any one of items 1-6, wherein the patient does not consume caffeine within 3 hours of administering the formulation comprising migalastat or a salt thereof.
8. The method of any one of items 1-7, wherein the therapeutically effective dose of migalastat or a salt thereof is in a range of from about 100 mg to about 150 mg every other day.
9. The method of any one of items 1-8, wherein the therapeutically effective dose of migalastat or a salt thereof is about 123 mg free base equivalent (FBE) every other day.
10. The method of any one of items 1-9, wherein the therapeutically effective dose of migalastat or a salt thereof is about 150 mg of migalastat hydrochloride every other day.
11. A method of treatment of Fabry disease in a human patient in need thereof, the method comprising orally administering to the patient a formulation comprising a therapeutically effective dose of migalastat or a salt thereof, wherein the patient does not consume caffeine within 2 hours of administering the formulation comprising migalastat or a salt thereof.
12. The method of item 11, further comprising administering caffeine to the patient at least about 2 hours prior to administering the formulation comprising migalastat or a salt thereof.
13. The method of item 11 or 12, further comprising administering caffeine to the patient at least about 2 hours after administering the formulation comprising migalastat or a salt thereof.
14. The method of any one of items 11-13, wherein the patient does not consume caffeine within 2 hours of administering the formulation comprising migalastat or a salt thereof to avoid a decrease in AUC and Cₘₐₓ for migalastat of about 57% and about 60%, respectively.
15. The method of any one of items 11-14, wherein the patient fasts for at least about 2 hours prior to administering the formulation comprising migalastat or a salt thereof and for at least about 2 hours after administering the formulation comprising migalastat or a salt thereof.
16. The method of any one of items 11-15, wherein the patient does not consume caffeine for at least about 3 hours prior to administering the formulation comprising migalastat or a salt thereof and for at least about 2 hours after administering the formulation comprising migalastat or a salt thereof.
17. The method of any one of items 11-16, wherein the patient does not consume caffeine within 3 hours of administering the formulation comprising migalastat or a salt thereof.
18. The method of any one of items 11-17, wherein the therapeutically effective dose of migalastat or a salt thereof is in a range of from about 100 mg to about 150 mg every other day.
19. The method of any one of items 11-18, wherein the therapeutically effective dose of migalastat or a salt thereof is about 123 mg free base equivalent (FBE) every other day.
20. The method of any one of items 11-19, wherein the therapeutically effective dose of migalastat or a salt thereof is about 150 mg of migalastat hydrochloride every other day.
21. Formulation comprising a therapeutically effective dose of migalastat or a salt thereof for use in the treatment of Fabry disease in a human patient in need thereof, wherein the formulation comprising migalastat or a salt thereof is administered without concurrent administration of caffeine.
22. The formulation according to item 21, wherein the formulation comprising migalastat or a salt thereof is administered orally to the patient.
23. The formulation according to any one of items 21 and 22, wherein the patient does not consume caffeine within a certain time interval of administering the formulation comprising migalastat or a salt thereof to avoid a decrease in AUC and Cₘₐₓ for migalastat of about 57% and about 60%, respectively.
24. The formulation according to any one of items 21 to 23, wherein the patient does not consume caffeine within a time interval from at least 30 minutes prior to, to at least 30 minutes after administering the formulation comprising migalastat or a salt thereof.
25. The formulation according to any one of items 21 to 24, wherein the patient does not consume caffeine within a time interval from at least 1 hour prior to, to at least 1 hour after administering the formulation comprising migalastat or a salt thereof.
26. The formulation according to any one of items 21 to 25, wherein the patient does not consume caffeine within a time interval from at least 2 hours prior to, to at least 2 hours after administering the formulation comprising migalastat or a salt thereof.
27. The formulation according to any one of items 21 to 26, wherein the patient does not consume caffeine within a time interval from at least 3 hours prior to, to at least 2 hours after administering the formulation comprising migalastat or a salt thereof.
28. The formulation according to any one of items 21 to 27, wherein the patient does not consume caffeine within a time interval from at least 3 hours prior to, to at least 3 hours after administering the formulation comprising migalastat or a salt thereof.
29. The formulation according to any one of items 21 to 28, wherein the patient consumes caffeine outside of the time interval for abstaining from caffeine.
30. The formulation according to any one of items 21 to 29, further comprising administering caffeine to the patient at least 4 hours prior to administering the formulation comprising migalastat or a salt thereof.
31. The formulation according to any one of items 21 to 30, further comprising administering caffeine to the patient at least 4 hours after administering the formulation comprising migalastat or a salt thereof.
32. The formulation according to any one of items 21 to 31, wherein the patient consumes non-caffeinated beverages during the time interval for abstaining from caffeine.
33. The formulation according to any one of items 21 to 32, wherein the patient fasts during the time interval for abstaining from caffeine.
34. The formulation according to any one of items 21 to 33, wherein the patient fasts within a time interval from at least 2 hours prior to, to at least 2 hours after administering the formulation comprising migalastat or a salt thereof.
35. The formulation according to any one of items 21 to 34, wherein the salt of migalastat is migalastat hydrochloride.
36. The formulation according to any one of items 21 to 35, wherein the therapeutically effective dose of migalastat or a salt thereof is in a range of from 100 mg to 150 mg every other day.
37. The formulation according to any one of items 21 to 36, wherein the therapeutically effective dose of migalastat or a salt thereof is about 123 mg free base equivalent (FBE) every other day.
38. The formulation according to any one of items 21 to 37, wherein the therapeutically effective dose of migalastat hydrochloride is about 150 mg every other day.

## Claims

1. Formulation comprising a therapeutically effective dose of migalastat or a salt thereof for use in a method of treatment of Fabry disease in a human patient in need thereof, the method comprising orally administering to the patient the formulation, wherein the patient does not consume caffeine within 2 hours of administering the formulation.

2. Formulation for use according to claim 1, further comprising administering caffeine to the patient at least about 2 hours prior to administering the formulation.

3. Formulation for use according to claim 1 or 2, further comprising administering caffeine to the patient at least about 2 hours after administering the formulation.

4. Formulation for use according to any one of claims 1-3, wherein the patient does not consume caffeine within 2 hours of administering the formulation to avoid a decrease in AUC and Cₘₐₓ for migalastat of about 57% and about 60%, respectively.

5. Formulation for use according to any one of claims 1-4, wherein the patient fasts for at least about 2 hours prior to administering the formulation and for at least about 2 hours after administering the formulation.

6. Formulation for use according to any one of claims 1-5, wherein the patient does not consume caffeine for at least about 3 hours prior to administering the formulation and for at least about 2 hours after administering the formulation.

7. Formulation for use according to any one of claims 1-6, wherein the patient does not consume caffeine within 3 hours of administering the formulation.

8. Formulation for use according to any one of claims 1-7, wherein the therapeutically effective dose of migalastat or a salt thereof is in a range of from about 100 mg to about 150 mg every other day.

9. Formulation for use according to any one of claims 1-8, wherein the therapeutically effective dose of migalastat or a salt thereof is about 123 mg free base equivalent (FBE) every other day.

10. Formulation for use according to any one of claims 1-9, wherein the therapeutically effective dose of migalastat or a salt thereof is about 150 mg of migalastat hydrochloride every other day.

11. Formulation for use according to any one of claims 1-10, wherein the patient consumes non-caffeinated beverages during the time interval that caffeine is not consumed.

12. Formulation for use according to any one of claims 1-11, wherein the formulation comprises an oral dosage form.

13. Formulation for use according to claim 12, wherein the oral dosage form comprises a tablet, a capsule or a solution.

14. Formulation for use according to any one of claims 1-13, wherein the patient is orally administered capsules each containing 150 mg migalastat hydrochloride or an equivalent dose of migalastat or a salt thereof other than the hydrochloride salt.

15. Formulation for use according to any one of claims 1-14, wherein the patient has a HEK assay amenable mutation in α-galactosidase A.
